# EUROPÄISCHE PATENTSCHRIFT

(11) **EP 1 771 200 B1**
(45) Veröffentlichungstag und Bekanntmachung des Hinweises auf die Patenterteilung: **08.06.2011**
(21) Anmeldenummer: 05769707.0
(22) Anmeldetag: 27.07.2005
(51) Int. Cl.: A61K 38/39, A61K 45/06, A61P 19/02, A61P 29/00

(54) **PHYSIOLOGISCH AKTIVE ZUSAMMENSETZUNG AUF KOLLAGENBASIS**
PHYSIOLOGICALLY-ACTIVE COMPOSITION BASED ON COLLAGEN
COMPOSITION PHYSIOLOGIQUEMENT ACTIVE A BASE DE COLLAGENE

(30) Priorität: 28.07.2004 DE 102004036577
(43) Veröffentlichungstag der Anmeldung: 11.04.2007
(73) Patentinhaber: Cargill, Incorporated, Wayzata, MN 55391 (US)
(72) Erfinder: PURPURA, Martin, 53227 Bonn (DE); JÄGER, Ralf, Milwaukee, WI 53202 (US); BALAN, Karim, 93055 Regensburg (DE); PAPER, Dietrich, 93142 Maxhütte-Haidhof (DE)
(74) Vertreter: Metten, Karl-Heinz
(86) Internationale Anmeldenummer: PCT/EP2005/008151
(87) Internationale Veröffentlichungsnummer: WO 2006/010606

(56) Entgegenhaltungen:
- EP-A- 0 577 143
- CA-A1- 2 212 649
- US-A1- 2001 048 952
- US-A1- 2002 086 070
- US-A1- 2003 069 171
- US-A1- 2004 086 581
- US-B1- 6 224 871
- DATABASE WPI Section Ch, Week 199937 Derwent Publications Ltd., London, GB; Class B04, AN 1999-442492 XP002352231 & RO 114 552 B1 (INST BIOLOGIA DEZVOLTARII BUCURESTI) 30. Juni 1999 (1999-06-30)
- DATABASE BIOSIS [Online] BIOSCIENCES INFORMATION SERVICE, PHILADELPHIA, PA, US; Juni 2002 (2002-06), GARBACKI NANCY ET AL: "Effects of prodelphinidins isolated from Ribes nigrum on chondrocyte metabolism and COX activity" XP002352229 Database accession no. PREV200200406523 & NAUNYN-SCHMIEDEBERG'S ARCHIVES OF PHARMACOLOGY, Bd. 365, Nr. 6, Juni 2002 (2002-06), Seiten 434-441, ISSN: 0028-1298

## Beschreibung

Gegenstand der vorliegenden Erfindung ist eine physiologisch aktive Zusammensetzung auf Kollagenbasis.

Bei Kollagen handelt es sich um ein natürliches Faserprotein, das den Hauptbestandteil des Stütz- und Bindegewebes bildet und sich insbesondere in der Haut, den Sehnen und Knochen findet. Kollagen stellt somit eines der wichtigsten Proteine des Bindegewebes dar und es macht ca. 30 % der gesamten Proteinmasse des Menschen aus. Kollagene gehören zu den wenigen Proteinen, deren Sequenz über längere Strecken periodisch aufgebaut ist und eine sehr starre Helix aus drei Ketten bildet. Allgemein unterscheidet man zwischen mehreren Kollagentypen, die sich im Aufbau und in der Sequenz unterscheiden, wobei das Kollagenmuster für das jeweilige Gewebe typisch ist. Der Kollagentyp I bspw. findet sich überwiegend in Knochen, Sehnen, in der Haut sowie in großen Blutgefäßen und der Cornea; der Kollagentyp II ist hauptsächlich an Rippenknorpeln und der Hornhaut beteiligt; die Kollagentypen III und IV schließlich bilden die großen Blutgefäße und die Haut bzw. sind sie an der Basalmembranbildung beteiligt.

Als körpereigene Substanz ist Kollagen seit langer Zeit im Zusammenhang mit der Behandlung von degenerativen Gelenkserkrankungen etabliert, wobei insbesondere die Behandlung von Arthrosen und arthritischen Formen im Vordergrund stehen.

Entzündungsprozesse und damit verbundene Schmerzformen sind bei degenerativen Gelenkserkrankungen das Ergebnis eines komplexen Wechselspiels verschiedener pathologischer Prozesse. Die Entstehung und Progression derartiger Entzündungs- und Schmerzzustände basiert fast immer auf der eigentlichen Entzündungsreaktion. Damit vergesellschaftet sind aber auch Veränderungen im Blutgefäßsystem und die aus diesen Veränderungen resultierenden Schwellungen, Rötungen und Erhitzungserscheinungen, aber auch Veränderungen der Empfindlichkeit der Schmerzrezeptoren bzw. Veränderungen bei der nervalen Schmerzweiterleitung und der Schmerzverarbeitung im zentralen Nervensystem sowie die Modulation des bzw. durch das Immunsystem spielen bei degenerativen Gelenkerkrankungen eine wichtige Rolle.

Dabei kann keines der genannten Felder als isoliert betrachtet werden, da bspw. die Freisetzung von bestimmten Entzündungsmediatoren sowohl zu einer Erweiterung der Gefäße und damit zu einer verstärkten Einwanderung von Zellen des Immunsystems als auch zu einer Herabsetzung der Schmerzschwelle führen. Die eingewanderten Immunzellen wiederum führten in der Folge durch den Abbau von Gewebsstrukturen und die Freisetzung von weiteren Entzündungsmediatoren zu einer Verstärkung des Entzündungsprozesses, wobei gleichzeitig freigesetzte Histaminmengen zu einer zusätzlichen Erhöhung der Durchblutung und zu Schmerz durch eine weitere Herabsetzung der Schmerzschwelle führen.

Eine zentrale Rolle im Geschehen degenerativer Gelenkerkrankungen und damit verbundener sekundärer Krankheitsformen spielen zentrale Mediatoren des Entzündungsgeschehens, wobei Prostaglandine und Leukotriene Schlüsselpositionen einnehmen, da Prostaglandine sowohl die Freisetzung lytischer Enzyme induzieren als auch das Entzündungsgeschehen verstärken, indem sie die Anzahl der Cytokin-Rezeptoren und damit die Reaktivität der beteiligten Zellen erhöhen. Gleichzeitig ist damit auch eine Herabsetzung der Schmerzschwelle verbunden. Ebenfalls beteiligt sind Cyclooxygenasen (COX-1 und COX-2), die Schlüsselreaktionen bei der Entstehung von Prostaglandinen aus Arachidonsäure katalysieren.

Wie die Prostaglandine entstehen auch Leukotriene aus Arachidonsäure, wobei die 5-Lipoxygenase eine wichtige Rolle spielt. Weitere Enzyme, die bei den Veränderungen im Gefäßsystem bzw. beim Schmerzgeschehen eine Rolle spielen, sind NO-Synthase, Histaminoxidase und Monooxygenasen sowie Oxidoreduktasen allgemeiner Art.

Aus dem Stand der Technik sind zahlreiche Literaturstellen bekannt, die Hinweise auf - Behandlungsmöglichkeiten von degenerativen Gelenkserkrankungen mit Hilfe von Kollagen geben.

In der US 6,224,871 B1 werden Nahrungsergänzungsmittel beschriehen, enthaltend enzymatisch hydrolisiertes Kollagen in Kombination mit Glycosaminsulfat und antiinflammatorisch oder anti-oxidativ wirkenden Verbindungen wie Ginkgo biloba, Curcuma, Aloe und weiteren Pflanzenextrakten. Dieses Präparat soll degenerative Gelenkserkrankungen mildern.

Aus der EP 577 143 A2 geht eine Flavonoidzubereitung hervor, enthaltend mindestens ein Flavonoid in molekular disperser Verteilung in einer Basissubstanz aus einem hydrophilen Peptid mit einem Molekulargewicht zwischen 100 D und 10⁸ D. Als hydrophiles Peptid kann auch ein Pflanzenproteinhydrolysat eingesetzt werden. Derartige Zubereitungen sollen es ermöglichen, das Flavonoid in gelöster (molekulardisperser) Form ohne die Anwesenheit eines organischen Lösungsmittels oder eines unerwünschte Nebenwirkungen verursachenden Lösungsvermittlers zu logern oder zu applizieren.

Die US 2003/0069171 A1 offenbart ein Nahrungsergänzungsmittel zur Behandlung von Bindegewebe, umfassend ein Glucoseaminsatz, Chondroitinsulfat, Kollagen und Natriumhyaluronat.

So beschreibt EP 0 254 289 B1 Mittel zur Behandlung von Arthrosen, die ein geschmacksloses oder geschmacksneutrales und enzymatisch hydrolysiertes Kollagen mit einem mittleren Molekulargewicht von 10 bis 80 kDalton enthalten. Das verwendete hydrolysierte Kollagen stammt dabei aus tierischer Haut, tierischen Knochen oder sonstigem ausreichend aufgereinigtem Bindegewebe. Die Hydrolyse des Kollagens erfolgt demgemäß hauptsächlich durch enzymatische Einflüsse, wobei die Bedingungen so gewählt werden, dass ein bestimmter Molekulargewichtsbereich erhalten wird. Je nach Herstellungsbedingungen weisen die Hydrolysate des Kollagens mehr oder weniger Carboxylgruppen bzw. Aminogruppen auf und sie besitzen unterschiedliche isoelektrische pH-Bereiche. Die unter diesem Schutzrecht eingesetzten hydrolysierten Kollagene werden bspw. unter der Produktbezeichnung "Gelita-Sol" (DGF Stoess AG) und "Arthred" (Degussa Food Ingredients GmbH) vermarktet.

Im Rahmen einer in EP 0 254 289 veröffentlichten Studie wurden bei Patienten mit Erkrankungen des degenerativen Formenkreises am Hüft- und/oder Kniegelenk hauptsächliche folgende positive Befunde festgestellt: Verbesserungen des Anlaufschmerzes und der Anlaufsteifheit, Abnahme von Belastungs- und Ermüdungsschmerzen sowie eine verminderte Druckdolenz über dem Gelenkspalt und ein verringerter Endphasenschmerz. Bezüglich der Beweglichkeit der Hüftgelenke wurde eine deutlich verbesserte Flexion festgestellt, was in noch ausgeprägterem Maße für die Beweglichkeit der Kniegelenke festgestellt werden konnte.

Aus US 6 211 143 ist ein Verfahren zur Steigerung der Knorpelmasse in Gelenken bekannt. Dabei wird hydrolysierte Gelatine mit einem Molekulargewicht zwischen 2 und 100 kDalton oral verabreicht. Zusätzlich kann den Tagesdosen an Gelatine auch noch mindestens ein Vertreter der Vitamin B-Gruppe und/oder eine organische oder anorganische Magnesiumverbindung zugesetzt werden.

Das Verfahren gemäß diesem US-Schutzrecht ist als Verbesserung der allgemein bekannten Verabreichung von enzymatisch hydrolysierter Gelatine zu betrachten, von dem bis zum damaligen Zeitpunkt lediglich Vermutungen zum präzisen Wirkungsmechanismus bekannt waren. So wurde angenommen, dass hydrolysiertes Kollagen die Symptome arthritischer Schädigungen verbessern konnte. Dabei wurde auch eine gewisse Toleranz gegenüber oral eingenommener Proteine vermutet. In diesem Zusammenhang wurde insbesondere in Abrede gestellt, dass durch die Einnahme von Vorläufern der Kollagensynthese wie bspw. hydrolysierter Gelatine größere Knorpelmassen gebildet werden könnten.

Bei "Gelatine" handelt es sich in der Regel um ein mit zumeist warmem Alkali behandeltes Kollagen, welches durch diese Maßnahme in Lösung gebracht werden kann. Gelatinelösungen bilden beim Abkühlen steife Gallerten. Bei Gelatinen handelt es sich somit um Proteine, die aus Kollagen gewonnen werden. Um bei der Verabreichung von Gelatinen möglicherweise auftretenden allergischen Reaktionen vorzubeugen, werden Gelatinen, die durch Extraktion aus Kollagen erhalten wurden, zusätzlich einer kontrollierten enzymatischen oder chemischen Hydrolyse unterworfen, um so definierte kurzkettige Peptide zu erhalten, die ihrerseits vollständig verstoffwechselt werden.

Ein weiteres Produkt, welches bei der Vorbeugung oder Behandlung von degenerativen Gelenkserkrankungen eingesetzt wird, ist "Arthred" (Degussa Food Ingredients GmbH). Bei diesem Produkt handelt es sich ebenfalls um ein enzymatisch hydrolysiertes Kollagen, bestehend aus kurzkettigen Peptiden mit einem niedrigen Gesamtmolekulargewicht von ca. 3 kDalton. Die in Arthred enthaltenen kollagenbasierten Peptide sind auf Grund der ausgewählten Molekulargewichte ausgezeichnet kaltwasserlöslich.

Wie bereits dargelegt, handelt es sich bei degenerativen Gelenkserkrankungen nicht zuletzt auch um entzündliche Prozesse. Zur Behandlung von Entzündungen, insbesondere auch im Bereich der Gelenke, bietet der Stand der Technik zahlreiche natürliche Präparate an. In diesem Zusammenhang kommen insbesondere Verbindungen mit anti-oxidativen Eigenschaften, wie sie bspw. Flavonoide zeigen, in Frage. Vor allem auch pflanzliche Extrakte, die mitunter eine Vielzahl unterschiedlicher Verbindungsklassen und Verbindungen mit anti-oxidativen Eigenschaften enthalten können, werden in vielfältiger Applikationsform angeboten. Ebenfalls von Interesse sind in diesem Zusammenhang die schon erwähnten Cyclooxygenasen, weshalb die entsprechenden COX-1 und COX-2 Inhibitoren bei entzündlichem Geschehen eine wichtige Rolle spielen. Erwähnenswert sind hier insbesondere Rutaecarpin, Kaempferol und Humolone.

Die nachfolgende Tabelle 1 nennt Pflanzen oder deren Teile mit ausgeprägter COX-2 inhibitorischer Aktivität und die dafür verantwortlichen Hauptinhaltsstoffe:

**Tabelle 1**

| **1.1 APIGENIN** |
|---|
| |
| Achillea millefolium L.- Milfoil, Yarrow; found in Plant Anisochilus carnosus WALL.- Panjiri-ka Pat; found in Plant Apium graveolens L.- Celery; found in Plant Araucaria bidwillii HOOK.- Monkey puzzle; found in Leaf Artemisia dracunculus L.- Tarragon; found in Plant Camellia sinensis (L.) KUNTZE- Tea; found in Leaf Centaurea calcitrapa L.- Star-Thistle; found in Plant Chamaemelum nobile (L.) ALL.- Garden Camomile, Perennial Camomile, Roman Camomile; found in Plant Colchicum autumnale L.- Autumn Crocus, Meadow Saffron; found in Tuber Conyza canadensis (L.) CRONQ.- Butterweed, Hogweed, Horseweed; found in Plant Coriandrum sativum L.- Chinese Parsley, Cilantro, Coriander; found in Fruit Daphne genkwa SIEB & ZUCC.- Yuan Hua; found in Flower Daucus carota L.- Carrot; found in Fruit Digitalis purpurea L.- Purple Foxglove; found in Flower Echinacea spp- Coneflower, Echinacea; found in Leaf Ginkgo biloba L.- Ginkgo, Maidenhair Tree; found in Leaf Glechoma hederacea L.- Alehoof; found in Plant Glycyrrhiza glabra L.- Commom Licorice, Licorice, Licorice-Root, Smooth Licorice; found in Root Hydnocarpus wightiana BLUME- Hindi Chaulmoogra; found in Seed |
| Jatropha gossypifolia-L.- Spanish Physic Nut; found in Leaf Linum usitatissimum L.- Flax, Linseed; found in Plant Lycopodium clavatum L.- Antler Herb, Clubmoss; found in Plant Marrubium vulgare L.- Horehound, White Horehound; found in Plant Matricaria recutita L.- Annual Camomile, German Camomile, Wild Camomile; found in Plant Mentha aquatica L.- Water Mint; found in Plant Mentha spicata L.- Hortela da Folha Miuda, Spearmint; found in Leaf Mentha x rotundifolia (L.) HUDSON- Applemint; found in Shoot Ocimum basilicum L.- Basil, Cuban Basil, Sweet Basil; found in Plant Olea europaea subsp. europaea- Olive; found in Leaf Origanum vulgare L.- Common Turkish Oregano, European Oregano, Oregano, Pot Marjoram, Wild Marjoram, Wild Oregano; found in Plant Passiflora incarnata L.- Manzana de Mayo, Mayapple, Passionflower; found in Plant Perilla frutescens (L.) BRITTON- Perilla; found in Seed Petroselinum crispum (MILLER) NYMAN EX A. W. HILLL- Parsley; found in Plant Phaseolus vulgaris subsp. var. vulgaris- Black Bean, Dwarf Bean, Field Bean, Flageolet Bean, French Bean, Garden Bean, Green Bean, Haricot, Haricot Bean, Haricot Vert, Kidney Bean, Navy Bean, Pop Bean, Popping Bean, Snap Bean, String Bean, Wax Bean; found in Plant Phoenix dactylifera L.- Date Palm; found in Stem Plantago major L.- Common Plantain; found in Leaf Pogostemon cablin (BLANCO) BENTH.- Patchouli; found in Plant Prosopis juliflora (SW.) DC.- Mesquite; found in Plant Prunus cerasus L.- Sour Cherry; found in Plant Rosmarinus officinalis L.- Rosemary; found in Plant Salix alba L.- White Willow; found in Bark Salvia officinalis L.- Sage; found in Plant Scutellaria galericulata L.- Marsh Skullcap; found in Plant Silybum marianum (L.) GAERTN.- Lady's Thistle, Milk Thistle; found in Fruit Tanacetum vulgare L.- Tansy; found in Plant Teucrium polium L.- Golden Germander; found in Plant Thymus serpyllum L.- Creeping Thyme; found in Plant Thymus vulgaris L.- Common Thyme, Garden Thyme, Thyme; found in Plant Triticum aestivum L.- Wheat; found in Seed |
| |

| **1.2 BAICALEIN** |
|---|
| |
| Plantago major L.- Common Plantain; found in Leaf Scutellaria baicalensis GEORGI- Baikal Skullcap, Chinese Skullcap, Huang Qin; found in Root Scutellaria galericulata L.- Marsh Skullcap; found in Plant Scutellaria sp ; found in Leaf |
| |

| **1.3 BERBERIN** |
|---|
| |
| Adonis vernalis L.- Spring Adonis; found in Plant Andira inermis HBK- Cabbage Bark; found in Bark Argemone mexicana L.- Prickly Poppy; found in Plant Berberis vulgaris L.- Barberry; found in Plant Chelidonium majus L.- Celandine; found in Plant Coptis chinensis FRANCH.- Chinese Goldthread, Huang-Lian, Huang-Lien; found in Rhizome Coptis japonica (THUNB.) MAKINO- Huang-Lia, Huang-Lian, Huang-Lien, Japanese Goldthread; found in Rhizome Coptis spp- Generic Goldthread; found in Rhizome Corydalis spp- Fumewort; found in Plant Eschscholzia californica subsp. californica- California Poppy; found in Shoot Hydrastis canadensis L.- Goldenseal; found in Root Macleaya cordata R. BROWN- Plume Poppy; found in Plant Mahonia aquifolium (PURSH) NUTT.- Blue Barberry, Holly Barberry, Holly Mahonia, Mountain Grape, Oregon Grape; found in Root Menispermum canadense L.- Moonseed; found in Plant Papaver somniferum L.- Opium Poppy, Poppyseed Poppy; found in Plant Phellodendron amurense RUPR.- Amur Cork Tree, Huang Bai, Huang Po, Po Mu; found in Bark Podophyllum hexandrum ROYLE- Himalayan Mayapple; found in Rhizome Podophyllum peltatum L.- Mayapple; found in Plant Sanguinaria canadensis L.- Bloodroot; found in Root Zanthoxylum alatum ROXB.- Indian Prickly Ash, Wartara Oil Tree; found in Bark Zanthoxylum americanum MILL.- Northem Prickly Ash; found in Plant |
| |

| **1.4 CINNAMALDEHYD** |
|---|
| |
| Cinnamomum verum J. PRESL- Ceylon Cinnamon, Cinnamon Bark 30,000 ppm Cinnamomum aromaticum NEES- Canela de la China (Sp.), Canelero chino (Sp.), Canelle de Cochinchine (Fr.), Cannelier Casse (Fr.), Cannelier de Chine (Fr.), Cassia, Cassia Bark, Cassia Lignea, China Junk Cassia, Chinazimt (Ger.), Chinese Cassia, Chinese Cinnamon, Chinesischer Zimtbaum (Ger.), Kashia-Keihi (Jap.), Saigon Cinnamon, Zimtcassie (Ger.) Bark 1,900 ppm Hyacinthus orientalis L.- Hyacinth Flower 6.9 ppm Syzygium aromaticum (L.) MERR. & L. M. PERRY- Clove, Clovetree Flower 1 ppm Cnicus benedictus L.- Blessed Thistle Plant Commiphora myrrha (NEES) ENGL.- African Myrrh, Herabol Myrrh, Mirra (Sp. It.), Myrrh, Myrrhe (Fr., Ger.), Somali Myrrh Resin, Exudate, Sap AYL Lavandula sp- Lavender Plant JBH Lycopersicon esculentum MILLER- Tomato Fruit Melaleuca bracteata F. VON MUELL.- Bracteate Tea-Tree Leaf GEO Myroxylon balsamum (L.) HARMS- Peru Balsam, Tolu Balsam Plant Pimenta dioica (L.) MERR.- Allspice, Clover-Pepper, Jamaica-Pepper, Pimenta, Pimento Plant Pogostemon cablin (BLANCO) BENTH.- Patchouli Plant Rosa damascena MILLER- Damask Rose Essential Oil Tamarindus indica L.- Indian Tamarind, Kilytree, Tamarind Fruit |
| |

| **1.5 CIRSILINEOL** |
|---|
| |
| Artemisia capillaris THUNB.- Capillary Wormwood; found in Plant Artemisia dracunculus L.- Tarragon; found in Plant Salvia officinalis L.- Sage; found in Plant Salvia tomentosa- Sage; found in Leaf Sideritis sp ; found in Leaf Thymus vulgaris L.- Common Thyme, Garden Thyme, Thyme; found in Leaf |
| |

| **1.6 CURCUMIN** |
|---|
| |
| Curcuma longa L.- Indian Saffron, Turmeric; found in Rhizome Curcuma xanthorrhiza ROXB.- Javan Turmeric, Temu Lawak; found in Rhizome Curcuma zedoaria (CHRISTM.) ROSCOE- Shoti, Zedoary; found in Rhizome Zingiber officinale ROSCOE- Ginger; found in Plant |
| |

| **1.7 EUGENOL** |
|---|
| |
| Acacia farnesiana (L.) WILLD.- Cassie, Huisache, Opopanax, Popinac, Sweet Acacia; found in Flower Achillea millefolium L.- Milfoil, Yarrow; found in Plant Acorus calamus L.- Calamus, Flagroot, Myrtle Flag, Sweet Calamus, Sweetflag, Sweetroot; found in Rhizome Ageratum conyzoides L.- Mexican ageratum; found in Shoot Alpinia galanga (L.) SW.- Greater Galangal, Languas, Siamese Ginger; found in Rhizome Alpinia officinarum HANCE- Chinese Ginger, Lesser Galangal; found in Rhizome Anethum graveolens L.- Dill, Garden Dill; found in Plant Apium graveolens L.- Celery; found in Plant Artemisia capillaris THUNB.- Capillary Wormwood; found in Essential Oil Artemisia dracunculus L.- Tarragon; found in Shoot |
| Boronia megastigma NEES ex BARTL.- Scented Boronia; found in Flower Calamintha nepeta subsp. glandulosa (REQ.) P.W.BALL- Turkish Calamint; found in Shoot Camellia sinensis (L.) KUNTZE- Tea; found in Fruit Cananga odorata (LAM.) HOOK. f. & THOMSON- Cananga, Ylang-Ylang; found in Flower Capsicum annuum L.- Bell Pepper, Cherry Pepper, Cone Pepper, Green Pepper, Paprika, Sweet Pepper; found in Fruit Cinnamomum aromaticum NEES- Canela de la China (Sp.), Canelero chino (Sp.), Canelle de Cochinchine (Fr.), Cannelier Casse (Fr.), Cannelier de Chine (Fr.), Cassia, Cassia Bark, Cassia Lignea, China Junk Cassia, Chinazimt (Ger.), Chinese Cassia, Chinese Cinnamon, Chinesischer Zimtbaum (Ger.), Kashia-Keihi (Jap.), Saigon Cinnamon, Zimtcassie (Ger.); found in Plant Cinnamomum camphora (L.) NEES & EBERM.- Camphor, Ho Leaf; found in Plant Cinnamomum verum J. PRESL- Ceylon Cinnamon, Cinnamon;. found in Bark Cistus ladaniferus L.- Ambreine, Gum Cistus, Labdanum, Rockrose; found in Leaf Coffea arabica L.- Coffee; found in Seed Commiphora myrrha (NEES) ENGL.- African Myrrh, Herabol Myrrh, Mirra (Sp. It.), Myrrh, Myrrhe (Fr., Ger.), Somali Myrrh; found in Resin, Exudate, Sap Croton eluteria (L.) SW.- Cascarilla; found in Bark Cuminum cyminum L.- Cumin; found in Fruit Curcuma longa L.- Indian Saffron, Turmeric; found in Essential Oil Cymbopogon winterianus JOWITT- Java Citronella, Mahapengiri; found in Plant Cynara cardunculus subsp. cardunculus- Artichoke; found in Essential Oil Daucus carota L.- Carrot; found in Seed Drimys winteri FORSTER & FORSTER f.- Winter's Bark; found in Bark Elsholtzia blanda BENTH.- Bantaluki, Bantulsi; found in Shoot Eucalyptus citriodora HOOK.- Citron-Scented Gum, Lemon Eucalyptus, Lemon-Scented Gum, Spotted Gum; found in Leaf Glycyrrhiza glabra L.- Commom Licorice, Licorice, Licorice-Root, Smooth Licorice; found in Root Helianthus annuus L.- Girasol, Sunflower; found in Flower Helichrysum angustifolium DC.- Everlasting, Immortelle; found in Plant |
| Humulus lupulus L.- Hops; found in Essential Oil Hyacinthus orientalis L.- Hyacinth; found in Flower Hyssopus officinalis L.- Hyssop; found in Flower, Leaf Iris x germanica L.- Orris; found in Rhizome Jasminum officinale L.- Jasmine, Poet's Jessamine; found in Flower Juglans regia L.- English Walnut; found in Leaf Lantana camara L.- Lantana, Wild Sage; found in Leaf Laurus nobilis L.- Bay, Bay Laurel, Bayleaf, Grecian Laurel, Laurel, Sweet Bay; found in Leaf Lavandula latifolia MEDIK.- Aspic, Broad-Leaved Lavender, Spike Lavender; found in Plant Lavandula x intermedia EMERIC ex LOIS- Dutch Lavender, Lavandin; found in Plant Levisticum officinale KOCH- Lovage; found in Root Ligustrum japonicum THUNB.- Japanese Privet, Ligustri Fructus; found in Flower Lycopersicon esculentum MILLER- Tomato; found in Fruit Magnolia kobus DC.- Hsin-I, Xin-Yi; found in Flower Melaleuca bracteata F. VON MUELL.- Bracteate Tea-Tree; found in Leaf Melaleuca viridiflora SOLAND.EX GAERTN.- Broad-Leaf Tea-Tree, Niaouli; found in Leaf Melia azedarach L.- Chinaberry; found in Wood Mentha arvensis var. piperascens MALINV. EX L. H. BAILEY- Cornmint, Field Mint, Japanese Mint; found in Essential Oil Mentha pulegium L.- European Pennyroyal; found in Plant Mentha spicata L.- Hortela da Folha Miuda, Spearmint; found in Leaf Mentha x piperita subsp. nothosubsp. piperita- Peppermint; found in Essential Oil Micromeria congesta BOISS. & HAUSSKN.- Kaya Yarpuzu; found in Leaf Micromeria fruticosa subsp. barbata (BOISS. & KY.) P.H. DAVIS- Tea Hyssop, Zopha, Zuta; found in Shoot Micromeria myrtifolia BOISS. & HOHEN- Dagcayi, Haydarotu, Topukcayi; found in Shoot Morus alba L.- Sang-Pai-Pi, White Mulberry; found in Plant Myristica fragrans HOUTT.- Mace, Muskatnussbaum (Ger.), Nutmeg, nogal moscado (Sp.), nuez moscada (Sp.); found in Seed |
| Myroxylon balsamum (L.) HARMS- Peru Balsam, Tolu Balsam; found in Plant Narcissus tazetta L.- Daffodil; found in Flower Nicotiana tabacum L.- Tobacco; found in Leaf Ocimum basilicum L.- Basil, Cuban Basil, Sweet Basil; found in Leaf, Plant Ocimum canum SIMS- Hoary Basil; found in Shoot Ocimum gratissimum L.- Agbo, Shrubby Basil; found in Plant, Seed , Shoot Ocimum kilimandscharicum GUERKE- African Blue Basil, Kenyan Perennial Basil; found in Flower, Shoot Ocimum sanctum L.- Holy Basil, Tulsi; found in Leaf Ocimum sp- Basil; found in Plant Ocimum suave WILLD.- Kenyan Tree Basil; found in Shoot Oenanthe javanica (BLUME) DC.- Javan Water Dropwort; found in Shoot Origanum majorana L.- Marjoram, Sweet Marjoram; found in Plant Origanum minutiflorum O. SCHWARZ & P.H. DAVIS- Small-Flowered Oregano; found in Shoot Origanum onites L.- Oregano, Pot Marjoram; found in Shoot Origanum vulgare L.- Common Turkish Oregano, European Oregano, Oregano, Pot Marjoram, Wild Marjoram, Wild Oregano; found in Shoot Pelargonium graveolens (L.) L'HER ex AIT.- Rose Geranium, Scented Geranium; found in Essential Oil Peumus boldus MOLINA- Boldo; found in Leaf Pimenta dioica (L.) MERR.- Allspice, Clover-Pepper, Jamaica-Pepper, Pimenta, Pimento; found in Fruit, Leaf Pimenta racemosa (MILL.) J. W. MOORE- Bayrum Tree, West Indian Bay; found in Leaf Pimpinella anisum L.- Anise, Sweet Cumin; found in Plant Piper betel L.- Betel Pepper; found in Leaf Piper nigrum L.- Black Pepper, Pepper, White Pepper; found in Fruit Pogostemon cablin (BLANCO) BENTH.- Patchouli; found in Plant Polianthes tuberosa L.- Tuberose; found in Flower Prunus cerasus L.- Sour Cherry; found in Plant Prunus dulcis (MILLER) D. A. WEBB- Almond; found in Flower Pycnanthemum setosum NUTT.- Setose Mountain Mint; found in Shoot |
| Rosa centifolia L.- Cabbage Rose; found in Essential Oil Rosa damascena MILLER- Damask Rose; found in Essential Oil Rosa gallica L.- French Rose; found in Flower Santalum album L.- White Sandalwood; found in Wood Sassafras albidum (NUTT.) NEES- Sassafras; found in Root Syzygium aromaticum (L.) MERR. & L. M.. PERRY- Clove, Clovetree; found in Flower, Leaf, Stern Thymus capitatus (L.) HOFFM.-'Sicilian' Thyme, Spanish Origanum, Spanish Thyme; found in Shoot Thymus cilicicus BOISS. & BAL.-'Anatolian' Thyme; found in Shoot Thymus funkii COUSS.- Funk's Thyme; found in Shoot Thymus vulgaris L.- Common Thyme, Garden Thyme, Thyme; found in Plant Tilia sp.- Basswood, Lime, Linden; found in Flower Trifolium pratense L.- Cowgrass, Peavine Clover, Purple Clover, Red Clover; found in Flower Umbellularia californica (HOOK. & ARN.) NUTT.- California Bay; found in Plant Vaccinium corymbosum L.- Blueberry; found in Fruit Vaccinium macrocarpon AITON- American Cranberry, Cranberry, Large Cranberry; found in Fruit Vanilla planifolia JACKS.- Bourbon Vanilla, Vanilla; found in Fruit Viola odorata L.- Common Violet, Sweet Violet; found in Flower, Leaf Zea mays L.- Corn; found in Seed |
| |

| **1.8 KAEMPFEROL** |
|---|
| |
| Abelmoschus moschatus MEDIK.- Ambrette, Musk, Okra, Muskmallow, Tropical Jewel Hibiscus; found in Flower Acacia catechu (L. f.) WILLD.- Black Cutch, Catechu; found in Plant Acacia farnesiana (L.) WILLD.- Cassie, Huisache, Opopanax, Popinac, Sweet Acacia; found in Plant Acacia senegal (L.) WILLD.- Gum Arabic, Gum Arabic Tree, Kher, Senegal Gum, Sudan Gum Arabic; found in Plant |
| Ageratum conyzoides L.- Mexican ageratum; found in Plant Allium cepa L.- Onion, Shallot; found in Bulb Allium sativum var. sativum L.- Garlic; found in Plant Allium schoenoprasum L.- Chives; found in Leaf Althaea officinalis L.- Marshmallow, White Mallow; found in Leaf Ammi visnaga (L.) LAM.- Visnaga; found in Plant Anethum graveolens L.- Dill, Garden Dill; found in Fruit Armoracia rusticana GAERTN. ET AL.- Horseradish; found in Leaf Asparagus officinalis L.- Asparagus; found in Root Azadirachta indica A. JUSS.- Neem; found in Flower Berberis vulgaris L.- Barberry; found in Plant Beta vulgaris subsp. subsp. vulgaris- Beet, Beetroot, Garden Beet, Sugar Beet; found in Plant Brassica oleracea var. botrytis 1. var. botrytis L.- Cauliflower; found in Flower, Leaf Brassica oleracea var. capitata 1. var. capitata L.- Cabbage, Red Cabbage, White Cabbage; found in Leaf Brassica oleracea var. gongylodes L.- Kohlrabi; found in Shoot Brassica oleracea var. sabellica 1. var. acephala DC- Curly Kale, Kale, Kitchen Kale, Scotch Kale; found in Leaf Calendula officinalis L.- Calendula, Pot-Marigold; found in Plant Camellia sinensis (L.) KUNTZE- Tea; found in Plant Capsicum frutescens L.- Cayenne, Chili, Hot Pepper, Red Chili, Spur Pepper, Tabasco; found in Anther Castanea sativa MILLER- European Chestnut; found in Leaf Catharanthus roseus (L.) G. DON- Madagascar Periwinkle, Rosy Periwinkle; found in Plant Ceiba pentandra (L.) GAERTN.- Kapok, Silk-Cotton Tree; found in Flower Centella asiatica (L.) URBAN- Gotu Kola, Pennywort; found in Plant Chimaphila umbellata (L.) NUTT.- King's Cure, Pipsissewa; found in Plant Cichorium intybus L.- Chicory, Succory, Witloof; found in Plant Cinnamomum camphora (L.) NEES & EBERM.- Camphor, Ho Leaf; found in Plant Citrus paradisi MacFAD.- Grapefruit; found in Fruit Cola acuminata (P. BEAUV.) SCHOTT & ENDL.- Abata Cola; found in Plant |
| Consolida ajacis (L.) SCHUR- Larkspur; found in Flower Cornus florida L.- American Dogwood; found in Flower Crocus sativus L.- Saffron; found in Flower Cucurbita pepo L.- Pumpkin; found in Leaf Cuscuta reflexa ROXB.- Amarbel; found in Plant Daucus carota L.- Carrot; found in Seed Diospyros virginiana L.- American Persimmon; found in Plant Dodonaea viscosa (L.) JACQ.- Hopwood; found in Plant Drimys winteri FORSTER & FORSTER f.- Winter's Bark; found in Leaf Echinacea spp- Coneflower, Echinacea; found in Leaf Elaeagnus angustifolia L.- Russian Olive, Silver Berry; found in Leaf Equisetum arvense L.- Field Horsetail, Horsetail; found in Plant Eriobotrya japonica (THUNB.) LINDL.- Loquat; found in Plant Erythroxylum coca var. coca- Coca; found in Plant Eupatorium perfoliatum L.- Boneset; found in Plant Euphorbia hirta L.- Queensland Asthma Herb; found in Leaf Euphorbia lathyris L.- Caper Spurge, Mole Plant; found in Leaf Ficus carica L.- Echte Feige (Ger.), Feigenbaum (Ger.), Fico (Ital.), Fig; Figueira (Port.), Figuier Commun (Fr.), Higo (Sp.), Higuera Comun (Sp.); found in Plant Foeniculum vulgare MILLER- Fennel; found in Plant Fragaria spp- Strawberry; found in Leaf Frangula alnus MILLER- Buckthom; found in Seed Geranium thunbergii SIEB. & ZUCC- Gennoshiouko, Oriental Geranium; found in Leaf Ginkgo biloba L.- Ginkgo, Maidenhair Tree; found in Leaf Glycine max (L.) MERR.- Soybean; found in Plant Glycyrrhiza glabra L.- Commom Licorice, Licorice, Licorice-Root, Smooth Licorice; found in Shoot Gossypium sp- Cotton; found in Flower Hamamelis virginiana L.- Witch Hazel; found in Leaf Harpagophytum procumbens (BURCH.) DC. EX MEISN.- Devil's Claw, Grapple Plant; found in Root Hippophae rhamnoides L.- Sallow Thorn, Sea Buckthorn, Yellow Spine; found in Fruit |
| Humulus lupulus L.- Hops; found in Leaf Hura crepitans L.- Sandbox Tree; found in Leaf Hydrangea arborescens L.- Hydrangea, Smooth Hydrangea; found in Root Isatis tinctoria L.- Dyer's Woad; found in Plant Juglans regia L.- English Walnut; found in Leaf Kalanchoe pinnata (LAM.) PERS.- Air Plant, Siempre Viva; found in Leaf Kalanchoe spathulata DC.- Beach Bells; found in Leaf Lactuca sativa L.- Lettuce; found in Plant Laurus nobilis L.- Bay, Bay Laurel, Bayleaf, Grecian Laurel, Laurel, Sweet Bay; found in Plant Ligustrum japonicum THUNB.- Japanese Privet, Ligustri Fructus; found in Flower Lycopersicon esculentum MILLER- Tomato; found in Seed Magnolia denudata DESR.- Hsin-I, Xin-Yi; found in Plant Magnolia kobus DC.- Hsin-I, Xin-Yi; found in Plant Magnolia officinalis REHDER & E. H. WILSON- Chinese Magnolia, Hou Pu, Magnolia-Bark; found in Plant Mangifera indica L.- Mango; found in Plant Matricaria recutita L.- Annual Camomile, German Camomile, Wild Camomile; found in Plant Melaleuca leucadendra (L.) L.- Cajeput; found in Plant Melia azedarach L.- Chinaberry; found in Plant Moringa oleifera LAM.- Ben Nut, Benzolive Tree, Drumstick Tree, Horseradish Tree, Jacinto (Sp.), Moringa, West Indian Ben; found in Flower Morus alba L.- Sang-Pai-Pi, White Mulberry; found in Wood Musa x paradisiaca L.- Banana, Plantain; found in Fruit, Plant Myristica fragrans HOUTT.- Mace, Muskatnussbaum (Ger.), Nutmeg, nogal moscado (Sp.), nuez moscada (Sp.); found in Plant Nelumbo nucifera L.- Water Lotus; found in Plant Nicotiana tabacum L.- Tobacco; found in Leaf Ocimum basilicum L.- Basil, Cuban Basil, Sweet Basil; found in Leaf Oenothera biennis L.- Evening-Primrose; found in Leaf Olea europaea subsp. europaea- Olive; found in Stem |
| Opuntia ficus-indica (L.) MILL.- Indian Fig, Nopal, Nopalito, Prickly Pear; found in Flower Origanum vulgare L.- Common Turkish Oregano, European Oregano, Oregano, Pot Marjoram, Wild Marjoram, Wild Oregano; found in Plant Paeonia lactiflora PALL.- Bai Shao (Chinese), Chih-Shao, Common Garden Peony, Peony, White Peony; found in Leaf Paeonia moutan- Moutan, Tree Peony; found in Leaf Paeonia suffruticosa ANDREWS- Moutan, Moutan Peony, Tree Peony; found in Leaf Panax ginseng C. A. MEYER- Chinese Ginseng, Ginseng, Korean Ginseng, Oriental Ginseng; found in Leaf Panax quinquefolius L.- American Ginseng, Ginseng; found in Plant Passiflora incarnata L.- Manzana de Mayo, Mayapple, Passionflower; found in Plant Pastinaca sativa L.- Parsnip; found in Seed Petasites japonicus (SIEBOLD & ZUCC.) MAXIM.- Butterbur; found in Plant Petroselinum crispum (MILLER) NYMAN EX A. W. HILLL- Parsley; found in Plant Phyllanthus emblica L.- Emblic, Myrobalan; found in Leaf Physalis peruviana L.- Cape Gooseberry, Ground Cherry; found in Fruit Pinus mugo TURRA- Dwarf Pine, Swiss Mountain Pine; found in Wood Pistacia lentiscus L.- Chios Mastictree, Lentisco (Sp.), Mastic, Mastictree, Mastixbaum (Ger.); found in Leaf Pisum sativum L.- Pea; found in Tissue Culture Plumeria acutifolia POIR.- Frangipani; found in Flower Podophyllum hexandrum ROYLE- Himalayan Mayapple; found in Rhizome Podophyllum peltatum L.- Mayapple; found in Rhizome Podophyllum pleianthum L.- Chinese Mayapple; found in Rhizome Polygonum hydropiper L.- Common Smartweed; found in Plant Polygonum hydropiperoides L.- Mild Water Pepper; found in Plant Populus tacamahacca MILL.- Balm Of Gilead; found in Plant Prunus armeniaca L.- Apricot; found in Leaf Prunus cerasus L.- Sour Cherry; found in Plant Prunus domestica L.- Plum; found in Wood Prunus dulcis (MILLER) D. A. WEBB- Almond; found in Plant Prunus laurocerasus L.- Cherry Laurel; found in Plant |
| Prunus persica (L.) BATSCH- Peach; found in Leaf Prunus serotina subsp. serotina- Black Cherry, Wild Cherry; found in Plant Prunus spinosa L.- Blackthom, Sloe; found in Flower Psidium cattleianum SABINE- Strawberry Guava; found in Plant Rhododendron dauricum L.- Chinese Alpenrose; found in Plant Rhus toxicodendron L.- Poison Ivy; found in Plant Ribes nigrum L.- Black Currant; found in Fruit Ricinus communis L.- Castorbean; found in Plant Robinia pseudoacacia L.- Black Locust; found in Flower Rosa damascena MILLER- Damask Rose; found in Plant Sambucus nigra L.- Black Elder, Elder, European Alder, European Elder, European Elderberry; found in Flower Sanguisorba minor SCOP.- Small Burnet; found in Plant Sanguisorba officinalis L.- Greater Burnet; found in Plant Schinus molle L.- California Peppertree, Mastic-Tree, Peruvian Peppertree; found in Leaf Schinus terebinthifolius RADDI- Brazilian Peppertree; found in Leaf Silybum marianum (L.) GAERTN.- Lady's Thistle, Milk Thistle; found in Seed Solanum tuberosum L.- Potato; found in Flower Sophora japonica L.- Japanese Pagoda Tree; found in Plant Spinacia oleracea L.-.Spinach; found in Plant Syzygium aromaticum (L.) MERR. & L. M. PERRY- Clove, Clovetree; found in Flower Tagetes erecta L.- Aztec Marigold, Marigold; found in Leaf Tagetes patula L.- French Marigold; found in Plant Terminalia catappa L.- Indian Almond, Malabar Almond, Tropical Almond; found in Leaf Teucrium polium L.- Golden Germander; found in Plant Theobroma cacao L.- Cacao; found in Leaf Thespesia populnea (L.) SOLAND.- Indian tulip tree; found in Flower Thevetia peruviana (PERS.) K. SCHUM.- Adelfa Amarilla (Sp.), Cabalonga (Sp.), Chirca (Sp.), Loandro-Amarelo (Port.), Luckynut, Oleandre Jaune (Fr.), Peruvian Yellow Oleander, Thevetie (Ger.), Yellow Oleander; found in Plant Thymus vulgaris L.- Common Thyme, Garden Thyme, Thyme; found in Plant Tribulus terrestris L.- Puncture-vine; found in Flower Trigonella foenum-graecum L.- Alholva (Sp.), Bockshornklee (Ger.), Fenugreek, Greek Clover, Greek Hay; found in Plant Tsuga canadensis (L.) CARRIERE- Eastem Hemlock; found in Branches Vaccinium vitis-idaea var. minus LODD.- Cowberry, Lingen, Lingonberry; found in Fruit Valeriana officinalis L.- Common Valerian, Garden-Heliotrope, Valerian; found in Plant Vicia faba L.- Broadbean, Faba Bean, Habas; found in Shoot Viola odorata L.- Common Violet, Sweet Violet; found in Plant Vitis vinifera L.- European Grape, Grape, Grapevine, Parra (Sp.), Vid (Sp.), Vigne Vinifere (Fr.), Weinrebe (Ger.), Wine Grape; found in Leaf Zingiber officinale ROSCOE- Ginger; found in Plant |
| |

| **1.9 OLEANOL-SÄURE** |
|---|
| |
| Achyranthes aspera BLUME- Chaff Flower; found in Plant Achyranthes bidentata BLUME- Chaff Flower; found in Fruit Akebia quinata (THUNB.) DECNE- Chocolate Vine; found in Stem Allium cepa L.- Onion, Shallot; found in Bulb Allium sativum var. sativum L.- Garlic; found in Plant Apocynum cannabinum L.- Bitterroot, Spreading Dogbane; found in Root Arctostaphylos uva-ursi (L.) SPRENGEL- Bearberry, Uva Ursi; found in Plant Calendula officinalis L.- Calendula, Pot-Marigold; found in Flower Catharanthus roseus (L.) G. DON- Madagascar Periwinkle, Rosy Periwinkle; found in Plant Centaurium erythraea RAFN.- Centaury; found in Plant Chenopodium album L.- Lambsquarter; found in Plant Citrullus colocynthis- Colocynth; found in Plant Cnicus benedictus L.- Blessed Thistle; found in Plant Cornus officinalis SIEB. & ZUCC.- Chinese Dogwood; found in Seed |
| Cyperus rotundus L.- Nutsedge; found in Tuber Daemonorops draco BL.- Dragon's Blood; found in Fruit Elaeagnus pungens THUNB.- Thomy Silver Berry; found in Leaf Eleutherococcus senticosus (RUPR. & MAXIM.) MAXIM.- Ci wu jia (Pinyin), Eleuthero Ginseng, Siberian Ginseng, Spiny Ginseng, Wu jia; found in Root Eriobotrya japonica (THUNB.) LINDL.- Loquat; found in Leaf Forsythia suspensa VAHL- Lian-Jiao, Lien-Chiao; found in Fruit Glechoma hederacea L.- Alehoof; found in Plant Harpagophytum procumbens (BURCH.) DC. EX MEISN.- Devil's Claw, Grapple Plant; found in Root Hedera helix L.- Ivy; found in Leaf Helianthus annuus L.- Girasol, Sunflower; found in Flower Humulus lupulus L.- Hops; found in Stem Hyssopus officinalis L.- Hyssop; found in Plant Lavandula latifolia MEDIK.- Aspic, Broad-Leaved Lavender, Spike Lavender; found in Leaf Leonurus cardiaca L.- Motherwort; found in Plant Ligustrum japonicum THUNB.- Japanese Privet, Ligustri Fructus; found in Fruit Ligustrum lucidum W. T. AITON- Chinese Privet, Glossy Privet, Ligustri Fructus, Privet, White Waxtree; found in Fruit Liquidambar orientalis MILLER- Oriental Storax, Oriental Styrax; found in Resin, Exudate, Sap Liquidambar styraciflua L.- American Styrax, Sweetgum; found in Resin, Exudate, Sap Luffa aegyptiaca MILLER- Luffa, Smooth Loofah, Vegetable Sponge; found in Seed Melaleuca leucadendra (L.) L.- Cajeput; found in Plant Melissa officinalis L.- Balm, Bee Balm, Lemonbalm, Melissa; found in Shoot Mentha spicata L.- Hortela da Folha Miuda, Spearmint; found in Leaf Mentha x rotundifolia (L.) HUDSON- Applemint; found in Tissue Culture Myristica fragrans HOUTT.- Mace, Muskatnussbaum (Ger.), Nutmeg, nogal moscado (Sp.), nuez moscada (Sp.); found in Seed Myroxylon balsamum (L.) HARMS- Peru Balsam, Tolu Balsam; found in Plant Nerium oleander L.- Oleander; found in Plant |
| Ocimum basilicum L.- Basil, Cuban Basil, Sweet Basil; found in Flower Ocimum suave WILLD.- Kenyan Tree Basil; found in Leaf Olea europaea subsp. europaea- Olive; found in Petiole Origanum majorana L.- Marjoram, Sweet Marjoram; found in Plant Origanum vulgare L.- Common Turkish Oregano, European Oregano, Oregano, Pot Marjoram, Wild Marjoram, Wild Oregano; found in Plant Panax ginseng C. A. MEYER- Chinese Ginseng, Ginseng, Korean Ginseng, Oriental Ginseng; found in Root Panax japonicus C.A.MEYER- Japanese Ginseng; found in Rhizome Panax quinquefolius L.- American Ginseng, Ginseng; found in Plant Phytolacca americana L.- Pokeweed; found in Root Plantago major L.- Common Plantain; found in Leaf Plectranthus amboinicus (LOUR.) SPRENGEL- Amboini Coleus, Country Borage, Cuban Oregano, French Thyme, Indian Borage, Mexican Mint, Soup Mint, Spanish Thyme; found in Plant Prunella vulgaris L.- Heal-All, Self-Heal; found in Plant Prunus cerasus L.- Sour Cherry; found in Fruit Psidium guajava L.- Guava; found in Leaf Quisqualis indica L.- Rangoon Creeper; found in Fruit Rosmarinus officinalis L.- Rosemary; found in Plant, Shoot Salvia officinalis L.- Sage; found in Leaf, Stem Salvia sclarea L.- Clary Sage; found in Plant Salvia triloba L.- Greek Sage; found in Plant Sambucus nigra L.- Black Elder, Elder, European Alder, European Elder, European Elderberry; found in Bark, Leaf Satureja hortensis L.- Summer Savory; found in Plant Satureja montana L.- Savory, Winter Savory; found in Plant Syzygium aromaticum (L.) MERR. & L. M. PERRY- Clove, Clovetree; found in Flower Thymus serpyllum L.- Creeping Thyme; found in Plant Thymus vulgaris L.- Common Thyme, Garden Thyme, Thyme; found in Plant Uncaria tomentosa DC- Cat's Claw, Garabato Amarillo, Una de Gato; found in Plant Vaccinium corymbosum L.- Blueberry; found in Plant Vaccinium myrtillus L.- Bilberry, Dwarf Bilberry, Whortleberry; found in Leaf Viburnum prunifolium L.- Black Haw; found in Bark Viscum album L.- European Mistletoe; found in Resin, Exudate, Sap Vitis vinifera L.- European Grape, Grape, Grapevine, Parra (Sp.), Vid (Sp.), Vigne Vinifere (Fr.), Weinrebe (Ger.), Wine Grape; found in Leaf Wax Zizyphus jujuba MILL.- Da-Zao, Jujube, Ta-Tsao; found in Plant |
| |

| **1.10 QUERCETIN** |
|---|
| |
| Abelmoschus esculentus (L.) MOENCH- Okra; found in Flower Abelmoschus moschatus MEDIK.- Ambrette, Musk Okra, Muskmallow, Tropical Jewel Hibiscus; found in Flower Acacia catechu (L. f.) WILLD.- Black Cutch, Catechu; found in Plant Acacia nilotica (L.) WILLD. ex DELILE- Babul; found in Plant Acacia senegal (L.) WILLD.- Gum Arabic, Gum Arabic Tree, Kher, Senegal Gum, Sudan Gum Arabic; found in Plant Achillea millefolium L.- Milfoil, Yarrow; found in Plant Actinidia chinensis PLANCHON- Kiwi; found in Plant Aesculus hippocastanum L.- Horse Chestnut; found in Bark Agathosma betulina (P. J. BERGIUS) PILLANS- Buchu, Honey Buchu, Mountain Buchu; found in Plant Ageratum conyzoides L.- Mexican ageratum; found in Plant Ailanthus altissima (MILL.) SWINGLE- Stinktree, Tree Of Heaven; found in Leaf Allium cepa L.- Onion, Shallot; found in Bulb Allium sativum var. sativum L.- Garlic; found in Bulb Allium schoenoprasum L.- Chives; found in Leaf Althaea officinalis L.- Marshmallow, White Mallow; found in Leaf Ammi majus L.- Bishop's Weed; found in Leaf Ammi visnaga (L.) LAM.- Visnaga; found in Plant Anastatica hierochuntica L.- Jericho Rose; found in Plant Anethum graveolens L.- Dill, Garden Dill; found in Plant Anogeissus latifolia WALL.- Gum Ghatti; found in Plant |
| Arachis hypogaea L.- Groundnut, Peanut; found in Plant Araucaria bidwillii HOOK.- Monkey puzzle; found in Flower Arctostaphylos uva-ursi (L.) SPRENGEL- Bearberry, Uva Ursi; found in Leaf Ardisia japonica L.- Marlberry; found in Plant Armoracia rusticana GAERTN. ET AL.- Horseradish; found in Leaf Artemisia dracunculus L.- Tarragon; found in Shoot Artocarpus altilis (PARKINS.) FOSBERG- Breadfruit; found in Leaf Asimina triloba (L.) DUNAL- Pawpaw; found in Leaf Asparagus officinalis L.- Asparagus; found in Root Avena sativa L.- Oats; found in Hay Azadirachta indica A. JUSS.- Neem; found in Flower, Leaf Barosma betulina (P. J. BERGIUS) BARTL. & H. L. WENDL.- Buchu; found in Plant Basella alba L.- Vinespinach; found in Plant Berberis vulgaris L.- Barberry; found in Plant Beta vulgaris subsp. subsp. vulgaris- Beet, Beetroot, Garden Beet, Sugar Beet; found in Plant Brassica oleracea var. botrytis 1. var. botrytis L.- Cauliflower; found in Flower, Leaf Brassica oleracea var, capitata 1. var. capitata L.- Cabbage, Red Cabbage, White Cabbage; found in Leaf Brassica oleracea var. gemmifera var. gemmifera DC- Brussel-Sprout, Brussels-Sprouts; found in Sprout Seedling Brassica oleracea var. gongylodes L.- Kohlrabi; found in Shoot Brassica oleracea var. sabellica 1. var. acephala DC- Curly Kale, Kale, Kitchen Kale, Scotch Kale; found in Leaf Caesalpinia pulcherrima (L.) SW.- Bird Of Paradise; found in Flower Calendula officinalis L.- Calendula, Pot-Marigold; found in Plant Camellia sinensis (L.) KUNTZE- Tea; found in Leaf, Plant Camptotheca acuminata DECAISNE- Happy Tree; found in Leaf Capparis spinosa L.- Caper, Caperbush; found in Flower Capsicum frutescens L.- Cayenne, Chili, Hot Pepper, Red Chili, Spur Pepper, Tabasco; found in Fruit Carum carvi L.- Caraway, Carum, Comino (Sp.), Comino de prado (Sp.), Kummel (Ger.); found in Fruit |
| Castanea sativa MILLER- European Chestnut; found in Bark, Leaf, Wood Catharanthus roseus (L.) G. DON- Madagascar Periwinkle, Rosy Periwinkle; found in Plant Cedrus deodora LOUD.- Deodar Cedar; found in Stem Bark Ceiba pentandra (L.) GAERTN.- Kapok, Silk-Cotton Tree; found in Leaf Centaurea calcitrapa L.- Star-Thistle; found in Plant Cichorium endivia L.- Endive, Escarole; found in Leaf Cichorium intybus L.- Chicory, Succory, Witloof; found in Plant Cinnamomum camphora (L.) NEES & EBERM.- Camphor, Ho Leaf; found in Plant Citrus limon (L.) BURMAN f.- Lemon; found in Flower Citrus paradisi MacFAD.- Grapefruit; found in Fruit Consolida ajacis (L.) SCHUR-Larkspur; found in Flower Coriandrum sativum L.- Chinese Parsley, Cilantro, Coriander; found in Fruit Coriaria myrtifolia L.- Mealy Tree; found in Leaf Coriaria thymifolia HUMB. & BONPL.- Ground Toot, Shanshi; found in Plant Cornus florida L.- American Dogwood; found in Flower Crataegus cuneata SIEB. & ZUCC.- Hawthorn; found in Fruit Crocus sativus L.- Saffron; found in Flower Cucurbita pepo L.- Pumpkin; found in Leaf Cymbopogon citratus (DC. ex NEES) STAPF- Lemongrass, West Indian Lemongrass; found in Plant Cytisus scoparius (L.) LINK.- Scotch Broom; found in Plant Daucus carota L.- Carrot; found in Seed Diospyros virginiana L.- American Persimmon; found in Plant Dodonaea viscosa (L.) JACQ.- Hopwood; found in Plant Drimys winteri FORSTER & FORSTER f.- Winter's Bark; found in Leaf Echinacea spp- Coneflower, Echinacea; found in Leaf Elaeagnus angustifolia L.- Russian Olive, Silver Berry; found in Leaf Eriobotrya japonica (THUNB.) LINDL.- Loquat; found in Plant Eucalyptus globulus LABILL.- Blue Gum, Eucalypt, Tasmanian Bluegum; found in Leaf Eupatorium perfoliatum L.- Boneset; found in Plant Euphorbia hirta L.- Queensland Asthma Herb; found in Plant |
| Euphorbia lathyris L.- Caper Spurge, Mole Plant; found in Leaf Fagopyrum esculentum MOENCH.- Buckwheat; found in Plant Ficus carica L.- Echte Feige (Ger.), Feigenbaum (Ger.), Fico (Ital.), Fig, Figueira (Port.), Figuier Commun (Fr.), Higo (Sp.), Higuera Comun (Sp.); found in Plant Filipendula ulmaria (L.) MAXIM.- Meadowsweet, Queen Of The Meadow; found in Flower Foeniculum vulgare MILLER- Fennel; found in Fruit Forsythia suspensa VAHL- Lian-Jiao, Lien-Chiao; found in Flower Fragaria spp- Strawberry; found in Leaf Geranium thunbergii SIEB. & ZUCC- Gennoshiouko, Oriental Geranium; found in Leaf Ginkgo biloba L.- Ginkgo, Maidenhair Tree; found in Leaf Glycine max (L.) MERR.- Soybean; found in Plant Glycyrrhiza glabra L.- Commom Licorice, Licorice, Licorice-Root, Smooth Licorice; found in Plant Gossypium sp- Cotton; found in Plant Haematoxylum campechianum L.- Campechy, Logwood; found in Leaf Hamamelis virginiana L.- Witch Hazel; found in Leaf Helianthus annuus L.- Girasol, Sunflower; found in Flower, Leaf Hibiscus rosa-sinensis L.- Chinese hibiscus, Shoe-flower; found in Plant Hippophae.rhamnoides L.- Sallow Thorn, Sea Buckthorn, Yellow Spine; found in Fruit Houttuynia cordata THUNB.- Dokudami, Fishwort, Yu Xing Cao; found in Plant Humulus lupulus L.- Hops; found in Plant Hydrangea arborescens L.- Hydrangea, Smooth Hydrangea; found in Root Hypericum perforatum L.- Common St. Johnswort, Goatweed, Hypericum, Klamath Weed, St. John's-wort; found in Plant Ipomoea batatas (L.) LAM- Sweet Potato; found in Root Isatis tinctoria L.- Dyer's Woad; found in Plant Juglans regia L.- English Walnut; found in Leaf Kalanchoe pinnata (LAM.) PERS.- Air Plant, Siempre Viva; found in Leaf Kalanchoe spathulata DC.- Beach Bells; found in Leaf Lactuca sativa L.- Lettuce; found in Plant |
| Laurus nobilis L.- Bay, Bay Laurel, Bayleaf, Grecian Laurel, Laurel, Sweet Bay; found in Leaf Ledum palustre L.- Marsh Tea, Wild Rosemary; found in Leaf Leonurus cardiaca L.- Motherwort; found in Plant Ligustrum japonicum THUNB.- Japanese Privet, Ligustri Fructus; found in Flower Lippia dulcis TREV.- Sweet Herb; found in Plant Ludwigia adscendens- Ascending ludwigia; found in Plant Ludwigia perennis L.- Perennial ludwigia; found in Plant Lycopersicon esculentum MILLER- Tomato; found in Fruit Magnolia denudata DESR.- Hsin-I, Xin-Yi; found in Plant Magnolia kobus DC.- Hsin-I, Xin-Yi; found in Plant Magnolia officinalis REHDER & E. H. WILSON- Chinese Magnolia, Hou Pu, Magnolia-Bark; found in Plant Malus domestica BORKH.- Apple; found in Pericarp Mangifera indica L.- Mango; found in Plant Matricaria recutita L.- Annual Camomile, German Camomile, Wild Camomile; found in Plant Melia azedarach L.- Chinaberry; found in Plant Moringa oleifera LAM.- Ben Nut, Benzolive Tree, Drumstick Tree, Horseradish Tree, Jacinto (Sp.), Moringa, West Indian Ben; found in Flower Morus alba L.- Sang-Pai-Pi, White Mulberry; found in Plant Musa x paradisiaca L.- Banana, Plantain; found in Fruit, Plant Myristica fragrans HOUTT.- Mace, Muskatnussbaum (Ger.), Nutmeg, nogal moscado (Sp.), nuez moscada (Sp.); found in Plant Nelumbo nucifera L.- Water Lotus; found in Flower Nerium oleander L.- Oleander; found in Leaf Nicotiana tabacum L.- Tobacco; found in Flower Ocimum basilicum L.- Basil, Cuban Basil, Sweet Basil; found in Leaf Oenothera biennis L.- Evening-Primrose; found in Herb, Leaf Olea europaea subsp. europaea- Olive; found in Stem Origanum vulgare L.- Common Turkish Oregano, European Oregano, Oregano, Pot Marjoram, Wild Marjoram, Wild Oregano; found in Plant |
| Paeonia lactiflora PALL.- Bai Shao (Chinese), Chih-Shao, Common Garden Peony, Peony, White Peony; found in Leaf Paeonia moutan- Moutan, Tree Peony; found in Leaf Paeonia suffruticosa ANDREWS- Moutan, Moutan Peony, Tree Peony; found in Leaf Panax notoginseng (BURKELL) HOO & TSENG- Sanchi Ginseng; found in Root Passiflora incarnata L.- Manzana de Mayo, Mayapple, Passionflower; found in Leaf Pastinaca sativa L.- Parsnip; found in Leaf Perilla frutescens (L.) BRITTON- Perilla; found in Leaf Persea americana MILLER- Avocado; found in Leaf Petroselinum crispum (MILLER) NYMAN EX A. W. HILLL- Parsley; found in Plant Phoenix dactylifera L.- Date Palm; found in Pollen Or Spore Phyllanthus niruri L.- Seed On The Leaf; found in Plant Pinus mugo TURRA- Dwarf Pine, Swiss Mountain Pine; found in Wood Pinus sylvestris L.- Scotch Pine; found in Bark Pistacia lentiscus L.- Chios Mastictree, Lentisco (Sp.), Mastic, Mastictree, Mastixbaum (Ger.); found in Leaf Plumeria acutifolia POIR.- Frangipani; found in Flower Podophyllum hexandrum ROYLE- Himalayan Mayapple; found in Rhizome Podophyllum peltatum L.- Mayapple; found in Resin, Exudate, Sap Podophyllum pleianthum L.- Chinese Mayapple; found in Rhizome Polygonum aviculare L.- Prostrate Knotweed; found in Plant Polygonum hydropiper L.- Common Smartweed; found in Plant Polygonum hydropiperoides L.- Mild Water Pepper; found in Plant Populus tacamahacca MILL.- Balm Of Gilead; found in Plant Prosopis juliflora (SW.) DC.- Mesquite; found in Plant Prunus armeniaca L.- Apricot; found in Plant Prunus cerasus L.- Sour Cherry; found in Fruit Prunus domestica L.- Plum; found in Plant Prunus dulcis (MILLER) D. A. WEBB- Almond; found in Plant Prunus laurocerasus L.- Cherry Laurel; found in Plant Prunus persica (L.) BATSCH- Peach; found in Plant Prunus serotina subsp. serotina- Black Cherry, Wild Cherry; found in Plant Prunus spinosa L.- Blackthorn, Sloe; found in Flower |
| Psidium cattleianum SABINE- Strawberry Guava; found in Plant Psidium guajava L.- Guava; found in Leaf Pteridium aquilinum (L.) KUHN- Bracken, Bracken Fern; found in Plant Pueraria montana subsp. var. lobata (WILLD.) MAESEN & S. M. ALMEIDA- Kudsu, Kudzu; found in Leaf Pyrus communis L.- Pear; found in Pericarp Quercus alba L.- White Oak; found in Bark Quercus infectoria OLIV.- Aleppo Oak, Dyer's Oak, Gall Oak; found in Leaf Quercus robur L.- English Oak; found in Bark Quercus velutina LAM.- Black Oak; found in Plant Rhododendron dauricum L.- Chinese Alpenrose; found in Plant Rhus toxicodendron L.- Poison Ivy; found in Plant Ribes nigrum L.- Black Currant; found in Fruit Ricinus communis L.- Castorbean; found in Plant Rosa damascena MILLER- Damask Rose; found in Flower Rosa spp- Rose Hips; found in Fruit Rumex acetosa L.- Garden Sorrel; found in Fruit Rumex crispus L.- Curly Dock, Lengua Die Vaca, Sour Dock, Yellow Dock; found in Leaf Ruta graveolens L.- Rue; found in Plant Salix alba L.- White Willow; found in Bark Sambucus nigra L.- Black Elder, Elder, European Alder, European Elder, European Elderberry; found in Leaf Sanguisorba minor SCOP.- Small Burnet; found in Plant Sanguisorba officinalis L.- Greater Burnet; found in Plant Schinus molle L.- California Peppertree, Mastic-Tree, Peruvian Peppertree; found in Leaf Schinus terebinthifolius RADDI- Brazilian Peppertree; found in Plant Senna occidentalis (L.) H. IRWIN & BARNEBY- Coffee Senna; found in Root Silybum marianum (L.) GAERTN.- Lady's Thistle, Milk Thistle; found in Seed Solanum tuberosum L.- Potato; found in Flower Solidago virgaurea L.- European Goldenrod, Woundwort; found in Leaf Sophora japonica L.- Japanese Pagoda Tree; found in Plant |
| Sorbus aucubaria L.- Rowan Berry; found in Fruit Spartium junceum L.- Genet, Spanish Broom, Weaver's Broom; found in Plant Spinacia oleracea L.- Spinach; found in Leaf Tagetes patula L.- French Marigold; found in Plant Tanacetum vulgare L.- Tansy; found in Plant Terminalia catappa L.- Indian Almond, Malabar Almond, Tropical Almond; found in Leaf Teucrium botrys L.- Field Germander; found in Plant Teucrium montanum L.- Mountain Germander; found in Plant Teucrium scordium- Water Germander; found in Plant Theobroma cacao L.- Cacao; found in Leaf Thespesia populnea (L.) SOLAND.- Indian tulip tree; found in Flower Thevetia peruviana (PERS.) K. SCHUM.- Adelfa Amarilla (Sp.), Cabalonga (Sp.), Chirca (Sp.), Loandro-Amarelo (Port.), Luckynut, Oleandre Jaune (Fr.), Peruvian Yellow Oleander, Thevetie (Ger.), Yellow Oleander; found in Plant Tilia sp.- Basswood, Lime, Linden; found in Flower Tribulus terrestris L.- Puncture-vine; found in Flower Tridax procumbens L.- Coatbuttons, Mexican daisy; found in Flower Trigonella foenum-graecum L.- Alholva (Sp.), Bockshornklee (Ger.), Fenugreek, Greek Clover, Greek Hay; found in Seed Triticum aestivum L.- Wheat; found in Plant Tussilago farfara L.- Coltsfoot; found in Leaf Uncaria catechu (L. f.) WILLD.- Gambir, Pale Catechu; found in Bulb Urginea maritima L.- European Squill; found in Bulb Vaccinium corymbosum L.- Blueberry; found in Plant Vaccinium macrocarpon AITON- American Cranberry, Cranberry, Large Cranberry; found in Fruit Vaccinium myrtillus L.- Bilberry, Dwarf Bilberry, Whortleberry; found in Leaf Vaccinium vitis-idaea var. minus LODD.- Cowberry, Lingen, Lingonberry; found in Leaf Valeriana officinalis L.- Common Valerian, Garden-Heliotrope, Valerian; found in Plant Viola odorata L.- Common Violet, Sweet Violet; found in Plant Viola tricolor L.- Pansy, Wild Violet; found in Flower Vitis vinifera L.- European Grape, Grape, Grapevine, Parra (Sp.), Vid (Sp.), Vigne - Vinifere (Fr.), Weinrebe (Ger.), Wine Grape; found in Fruit Zea mays L.- Corn; found in Plant Zingiber officinale ROSCOE- Ginger; found in Plant |
| |

| **1.11 RESVERATROL** |
|---|
| |
| Morus alba L.- Sang-Pai-Pi, White Mulberry; found in Wood Polygonum cuspidatum SIEBOLD & ZUCC.- Giant Knotweed, Hu-Zhang, Japanese Knotweed, Mexican Bamboo; found in Root Vitis vinifera L.- European Grape, Grape, Grapevine, Parra (Sp.), Vid (Sp.), Vigne Vinifere (Fr.), Weinrebe (Ger.), Wine Grape; found in Leaf |
| |
| Trans-Resveratrol: Arachis hypogaea L.- Groundnut, Peanut; found in Sprout Seedling |
| |

| **1.12 SALICYL-SÄURE** |
|---|
| |
| Abies alba MILLER- Silver-Fir; found in Plant Acacia farnesiana (L.) WILLD.- Cassie, Huisache, Opopanax, Popinac, Sweet Acacia; found in Plant Achillea millefolium L.- Milfoil, Yarrow; found in Plant Althaea officinalis L.- Marshmallow, White Mallow; found in Leaf Anacardium occidentale L.- Cashew; found in Fruit Arachis hypogaea L.- Groundnut, Peanut; found in Seed Artemisia absinthium L.- Wormwood; found in Plant Artemisia dracunculus L.- Tarragon; found in Leaf Beta vulgaris subsp. subsp. vulgaris- Beet, Beetroot, Garden Beet, Sugar Beet; found in Root |
| Bixa orellana L.- Achiote, Annato, Annatto, Annoto, Arnato, Bija, Lipstick Pod, Lipsticktree; found in Plant Brassica oleracea var. botrytis 1. var. botrytis L.- Cauliflower; found in Leaf Calea zacatechichi SCHLECHT.- Bitter Grass, Dog's Grass, Mexican Calea; found in Plant Calendula officinalis L.- Calendula, Pot-Marigold; found in Plant Camellia sinensis (L.) KUNTZE- Tea; found in Leaf Cananga odorata (LAM.) HOOK. f. & THOMSON- Cananga, Ylang-Ylang; found in Flower Cimicifuga racemosa (L.) NUTT.- Black Cohosh, Black Snakeroot; found in Plant Cinnamomum aromaticum NEES- Canela de la China (Sp.), Canelero chino (Sp.), Canelle de Cochinchine (Fr.), Cannelier Casse (Fr.), Cannelier de Chine (Fr.), Cassia, Cassia Bark, Cassia Lignea, China Junk Cassia, Chinazimt (Ger.), Chinese Cassia, Chinese Cinnamon, Chinesischer Zimtbaum (Ger.), Kashia-Keihi (Jap.), Saigon Cinnamon, Zimtcassie (Ger.); found in Plant Colchicum autumnale L.- Autumn Crocus, Meadow Saffron; found in Plant Cucurbita pepo L.- Pumpkin; found in Seed Dipteryx odorata (AUBL.) WILLD.- Dutch Tonka Bean, Tonka Bean; found in Leaf Filipendula ulmaria (L.) MAXIM.- Meadowsweet, Queen Of The Meadow; found in Flower Fragaria spp- Strawberry; found in Fruit Gloriosa superba L.- Glory Lily; found in Bulb Glycine max (L.) MERR.- Soybean; found in Seed Glycyrrhiza glabra L.- Commom Licorice, Licorice, Licorice-Root, Smooth Licorice; found in Root Gossypium sp- Cotton; found in Root Hedeoma pulegioides (L.) PERS.- American Pennyroyal; found in Plant Iris versicolor L.- Blue Flag; found in Rhizome Jasminum officinale L.- Jasmine, Poet's Jessamine; found in Leaf Matricaria recutita L.- Annual Camomile, German Camomile, Wild Camomile; found in Plant Mentha pulegium L.- European Pennyroyal; found in Essential Oil Panax quinquefolius L.- American Ginseng, Ginseng; found in Plant |
| Pisum sativum L.- Pea; found in Plant Plantago major L.- Common Plantain; found in Plant Polygonum aviculare L.- Prostrate Knotweed; found in Plant Polypodium vulgare L.- Common Polypody, Sweet Fern; found in Leaf Ribes rubrum L.- Red Currant, White Currant; found in Fruit Rosa multiflora THUNB. ex MURRAY- Multiflora Rose; found in Fruit Rubus idaeus L.- Raspberry, Red Raspberry; found in Fruit Salix alba L.- White Willow; found in Plant Salvia officinalis L.- Sage; found in Plant Stachytarpheta cayennensis VAHL- Verbena; found in Plant Trifolium pratense L.- Cowgrass, Peavine Clover, Purple Clover, Red Clover; found in Flower Triticum aestivum L.- Wheat; found in Seed Vaccinium vitis-idaea var. minus LODD.- Cowberry, Lingen, Lingonberry; found in Fruit Viburnum prunifolium L.- Black Haw; found in Bark Viola odorata L.- Common Violet, Sweet Violet; found in Leaf Viola tricolor L.- Pansy, Wild Violet; found in Flower Vitis vinifera L.- European Grape, Grape, Grapevine, Parra (Sp.), Vid (Sp.), Vigne Vinifere (Fr.), Weinrebe (Ger.), Wine Grape; found in Root |
| |

| **1.13 URSOLIN-SÄURE** |
|---|
| |
| Agrimonia eupatoria L.- Agrimony, Sticklewort; found in Plant Arbutus unedo L.- Arbutus, Strawberry Tree; found in Leaf Arctostaphylos uva-ursi (L.) SPRENGEL- Bearberry, Uva Ursi; found in Leaf Artocarpus heterophyllus LAM.- Jackfruit; found in Root Catalpa bignonioides WALT.- Indian bean; found in Leaf Catharanthus roseus (L.) G. DON- Madagascar Periwinkle, Rosy Periwinkle; found in Leaf Chimaphila umbellata (L.) NUTT.- King's Cure, Pipsissewa; found in Plant Cornus florida L.- American Dogwood; found in Plant |
| Cornus officinalis SIEB. & ZUCC.- Chinese Dogwood; found in Fruit, Seed Crataegus cuneata SIEB. & ZUCC.- Hawthorn; found in Fruit Crataegus laevigata (POIR.) DC- English Hawthorn, Hawthorn, Whitethorn, Woodland Hawthorn; found in Leaf Cryptostegia grandifolia R. BR.- Rubber Vine; found in Leaf Elaeagnus pungens THUNB.- Thorny Silver Berry; found in Leaf Eriobotrya japonica (THUNB.) LINDL.- Loquat; found in Leaf Eucalyptus citriodora HOOK.- Citron-Scented Gum, Lemon Eucalyptus, Lemon-Scented Gum, Spotted Gum; found in Plant Forsythia suspensa VAHL- Lian-Jiao, Lien-Chiao; found in Fruit Gaultheria fragrantissima WALL.- Indian Wintergreen; found in Leaf Glechoma hederacea L.- Alehoof; found in Plant Helichrysum angustifolium DC.- Everlasting, Immortelle; found in Flower, Stem Humulus lupulus L.- Hops; found in Stem Hyssopus officinalis L.- Hyssop; found in Plant Ilex paraguariensis ST. HIL.- Mate, Paraguay Tea, South American Holly; found in Leaf Lavandula angustifolia MILLER- English Lavender; found in Leaf Lavandula latifolia MEDIK.- Aspic, Broad-Leaved Lavender, Spike Lavender; found in Leaf Leonurus cardiaca L.- Motherwort; found in Plant Ligustrum japonicum THUNB.- Japanese Privet, Ligustri Fructus; found in Fruit Limonia acidissima L.- Elephant Apple, Manzana De Elefante, Wood-Apple; found in Wood Lycopus europeus L.- European Bugle; found in Plant Malus domestica BORKH.- Apple; found in Fruit Epidermis Marrubium vulgare L.- Horehound, White Horehound; found in Plant Melaleuca leucadendra (L.) L.- Cajeput; found in Plant Melissa officinalis L.- Balm, Bee Balm, Lemonbalm, Melissa; found in Plant Mentha spicata L.- Hortela da Folha Miuda, Spearmint; found in Leaf Mentha x rotundifolia (L.) HUDSON- Applemint; found in Tissue Culture Monarda didyma L.- Beebalm, Oswego Tea; found in Leaf Nerium oleander L.- Oleander; found in Plant |
| Ocimum basilicum L.- Basil, Cuban Basil, Sweet Basil; found in Flower, Leaf , Sprout Seedling , Stem Ocimum canum SIMS- Hoary Basil; found in Shoot Origanum majorana L.- Marjoram, Sweet Marjoram; found in Plant Origanum vulgare L.- Common Turkish Oregano, European Oregano, Oregano, Pot Marjoram, Wild Marjoram, Wild Oregano; found in Plant Plantago asiatica L.- Asian Plantain; found in Plant Plantago major L.- Common Plantain; found in Plant Plectranthus amboinicus (LOUR.) SPRENGEL- Amboini Coleus, Country Borage, Cuban Oregano, French Thyme, Indian Borage, Mexican Mint, Soup Mint, Spanish Thyme; found in Plant Prunella vulgaris L.- Heal-All, Self-Heal; found in Plant Prunus cerasus L.- Sour Cherry; found in Fruit Prunus laurocerasus L.- Cherry Laurel; found in Leaf Prunus persica (L.) BATSCH- Peach; found in Leaf Prunus serotina subsp. serotina- Black Cherry, Wild Cherry; found in Leaf Psidium guajava L.- Guava; found in Leaf Punica granatum L.- Granado (Sp.), Granatapfelbaum (Ger.), Granatapfelstrauch (Ger.), Grenadier (Fr.), Mangrano (Sp.), Pomegranate, Romanzeiro (Port.), Zakuro (Jap.); found in Fruit, Leaf Pyrus communis L.- Pear; found in Fruit Rhododendron dauricum L.- Chinese Alpenrose; found in Plant Rhododendron ferrugineum L.- Rusty-Leaved Alpenrose; found in Leaf Rhododendron ponticum L.- Pontic Alpenrose; found in Leaf Rosmarinus officinalis L.- Rosemary; found in Plant, Shoot Rubus fruticosus- Blackberry; found in Plant Salvia officinalis L.- Sage; found in Leaf, Stem Salvia sclarea L.- Clary Sage; found in Plant Salvia triloba L.- Greek Sage; found in Plant Sambucus nigra L.- Black Elder, Elder, European Alder, European Elder, European Elderberry; found in Bark, Flower , Leaf Sanguisorba officinalis L.- Greater Burnet; found in Plant Satureja hortensis L.- Summer Savory; found in Leaf, Stem |
| Satureja montana L.- Savory, Winter Savory; found in Plant Sorbus aucubaria L.- Rowan Berry; found in Fruit Syringa vulgaris L.- Lilac; found in Leaf Teucrium chamaedrys L.- Wall Germander; found in Plant Teucrium polium L.- Golden Germander; found in Plant Teucrium scordium- Water Germander; found in Plant Teucrium scorodonia L.- Germander, Wood Germander; found in Leaf Thevetia peruviana (PERS.) K. SCHUM.- Adelfa Amarilla (Sp.), Cabalonga (Sp.), Chirca (Sp.), Loandro-Amarelo (Port.), Luckynut, Oleandre Jaune (Fr.), Peruvian Yellow Oleander, Thevetie (Ger.), Yellow Oleander; found in Leaf Thymus serpyllum L.- Creeping Thyme; found in Plant Thymus vulgaris L.- Common Thyme, Garden Thyme, Thyme; found in Plant Uncaria tomentosa DC- Cat's Claw, Garabato Amarillo, Una de Gato; found in Plant Vaccinium corymbosum L.- Blueberry; found in Plant Vaccinium myrtillus L.- Bilberry, Dwarf Bilberry, Whortleberry; found in Fruit, Leaf Vaccinium vitis-idaea var. minus LODD.- Cowberry, Lingen, Lingonberry; found in Fruit Verbena officinalis L.- Vervain; found in Plant Viburnum opulus subsp. var. opulus- Crampbark, European Cranberry Bush, Guelder Rose, Snowballbush; found in Fruit Viburnum prunifolium L.- Black Haw; found in Bark Vinca minor L.- Periwinkle, Running-Myrtle; found in Leaf, Plant Zizyphus jujuba MILL.- Da-Zao, Jujube, Ta-Tsao; found in Plant |

Die bekanntesten Vertreter des Pflanzenreiches mit ausgewiesener COX-2 hemmender Aktivität sind Aloe, Grüntee-Extrakte, Johannisbeere und Süßkirsche, Scilla, Ingwer, Curucuma und Ginkgo.

Da von Kollagen und dessen Hydrolyseprodukt Gelatine nur eine verschwindend geringe anti-inflammatorische Wirkung bekannt ist, hat sich für die vorliegende Erfindung die Aufgabe gestellt, eine neue physiologisch aktive Zusammensetzung auf Kollagenbasis bereitzustellen, die neben der bekannt guten Wirkung von Kollagen insbesondere auf degenerative Gelenkserkrankungen auch die Eigenschaft besitzt, positiv auf entzündliche Vorgänge vor allem im Gelenksbereich zu wirken bzw. derartige positive Auswirkungen zu unterstützen. Dabei stand das Ziel im Vordergrund, mit der neuen Zusammensetzung eine Jeicht applizierbare Variante zur Verfügung zu stellen, die hinsichtlich der Compliance keinerlei Probleme aufwirft.

Zudem durften die aktiven Hestandteile zu keinerlei negativen Begleiterscheinungen führen, weshalb sie insbesondere auf natürliche Quellen zurückzuführen sein sollten. Auch war eine etwaige negative Wechselwirkung zwischen den physiologisch aktiven Hauptbestandteilen zu vermeiden, so dass sowohl die Kollagenkomponente als auch die weiteren Bestandteile mit den gewünschten physiologischen Aktivitäten voll der Vorbeugung und Behandlung insbesondere degenerativer Gelenkserkrankungen zugute kommen.

Gelöst wurde diese Aufgabe mit einer Zusammensetzung gemäß den Merkmalen des Patentanspruchs 1.

Vollkummen überraschend wurde bei der Austestung repräsentativer erfindungsgemäßer Zusammensetzungen festgestellt, dass die bekannt gute Wirkung der Kollagenkompoponente in der beanspruchten Zusammensetzung keinerlei Abschwächung erlitt, sondern dass vor allem die gelensschützende/-stabilisierende Wirkung des Kollagens durch die weitere physiologisch aktive Kumponente II nicht nur additiv, sondern deutlich synergistisch gesteigert werden konnte. So konnte, hauptsächlich bedingt durch die Kollagenkomponente, nicht nur der Funktionsstatus erhtalten bzw. erhöht werden, sondern der Schmerzzustand kann durch die Veräbreichung der beanspruchten Zusammensetzung bei chronisch-entzündlichen Prozessen signifikant verbessert werden. Diese synergistische Wirkung der beiden Komponenten I und II geht eindeutig über die Wirkung der Einzelsubstanzen hinaus, was in diesem Ausmaß nicht vorherzusehen war.

Im Hinblick auf die Komponente 1 hat es sich im Rahmen der vorliegenden Erfindung als günstig erwiesen, wenn diese in mittleres Molekulargewicht von 1 bis 10 und besonders bevorzugt von 2 bis 6 kDalton aufweist. Ganz besonders bevorzugt wird eine Komponente 1 mit einem miltleren Molekulargewicht von 2 bis 4 kDalton. Gut geeignet im Sinn der vorliegenden Erfindung ist auch eine Komponente 1 mit tierischem Ursprung, wobei das Kollagen bspw. von Rindern oder Schweinen und aus deren Knochen, Häuten und Bindegeweben stammt.

Da mit der vorliegenden Zusammensetzung keinerlei Beschränkung im Hinblick auf eine spezifische Applikationsform verbunden sein sollte, hat es sich als empfehlenswert herausgestellt, in der erfindungsgemäßen Zusummensetzung als Komponente 1 ein kaltwasserlösliches Kollagen einzusetzen.

Die vorliegende Erfindung sieht im Zusammenhang mit der Komponente II vor, dass diese biologischen Ursprungs ist, wobei Varianten auf Basis einer Fermentationsbrühe und/oder eines Extraktes als bevorzugt anzusehen sind. Die Fermentationsbrühe kann dabei auch in unbehandelter Form cingesetzt, wenn die darin enthaltenen Verbindungen mit anti-oxidativen und/oder anti-inflammatorischen Eigenschaften in wirksamen Mengen enthalten sind. Bei den ebenfalls bevorzugten Extrakten kann es sich um flüssige Varianten hundeln, die entweder als wässrige Auszüge oder alkoholische Produkte vorliegen oder aber aus diesen durch Trocknungsvorgänge in Pulverform hervorgegangen sind. Hinsichtlich der Extrakte als bevorzugte Variante für Komponente II werden von der vorliegenden Erfindung Extrakte pflanzlichen Ursprungs als besonders bevorzugt umfasst.

Aus Gründen der Verabreichung und Compliance wird es im Rahmen der Erfindung als bevorzugt angesehen, wenn es sich hei der Komponente II um einen Extrakt, ein Lyophilisat und/oder eine Fraktion handelt. wobei diese drei Darreichungsvarianten insbesondere von Plantago spec., wie Plantigo major, Plantago media oder Plantago Lanccolata, von Aloe vera, Atoysia triphylla, Humulus lupulus, Ginko biloba, Lippia triphylla und/oder Lippia citriodora, stammen sollten.

Die zuletzt genannten Pflanzenspezies bieten sich deshalb im vorliegenden Zusammenhang besonders an, da deren aktive Inhaltsstoffe Flavonoide umfassen bzw. anti-oxidative Eigenschaften in Form von Troloxäquivalenten zeigen und somit als Matrixprotektoren bei entzündlichen Krankheitsformen, die ggf. durch reaktive Sauerstoffspezies ausgelöst wurden oder verstärkt werden, eingesetzt werden können.

Insbesondere Extrakte der als Verbenenkraut bekannten Aloysia triphylla werden in Frankreich traditionell zur symptomatischen Behandlung von Verdauungsbeschwerden einerseits, aber auch Nervosität und Schlafstörungen andererseits angewendet.

Aus der Vielzahl der für Komponente II in Frage kommender Verbindungen und Verbindungsklassen mit anti-oxidativen und/oder anti-inflammatorischen Eigenschaften hahen sich für die beanspruchte Zusammensetzung insbesondere solche als geeignet erwiesen, die Phenylethanoide, umfassend Acteoside, und genebenenfalls einen COX-2 Inhibitor wie z.B. die aus Hopfen bekannten α- und β-Säuren. Apigenin, Baicalein, Berberin, Cinnamaldehyd, Cirsilincol, Curcumin, Eugenol, Kaempferol, Oleandsäure, Querectin, Resveratol, Salicylsäure und Ursolinsäure enthalten.

Spitzwegericharten (Plantago spec.) enthalten als aktive Inhaltsstoffe Iridoidglykoside, Flavone, Phenolcarbonsäuren, Cumarine, Phenylethanoide und Polysaccharide. Aus der Verbindungsklasse der Flavone wurden die Glykoside Apigenin-7-O-Glukosid. Apigenin-6,8-di-C-Glukosid, Apigenin-7-O-Glukuronid, Apigenin-7-O-Glukuronylglukosid, Luteolin-7-O-Glukosid, Luteolin-7-O-Glukuronid, Lutcolin-7-O-Glukuronid-3'-O-Glukosid sowie Luteolin-7-O-Glukuronylglukosid ermittelt. Prominente Vertreter der Phenolcarbonsäuren sind in Plantago Chlorogensäure und Cistanoid sowie 4-Hydroxybenzoesäure, Protocatechusäure. Gentisinsäure usw., ebenfalls enthalten ist Aescolitin als typisches Cumarin. Bei den ebenfalls in Plantago enthaltenen Phenylethanoiden handelt es sich um eine Inhaltsstoffgruppe, deren wesentliches Strukturmerkmal in der Ausbildung eines Vollacetals aus Glukose und Phenylethanol sowie eines Esters von Kaffreesäure mit Glukose besteht. Aus Plantago lanceolata bspw. wurden ca. 3,5% Acteosid (Verbascosid) und ca. 1% Plantamajosid isoliert. Weitere Phenylethanoide stellen Isoacteosid und Lavandulifoliosid dar.

Die bereits genannten Acteoside sind natürlich vorkommende Disaccharide mit akut und chronisch anti-inflammatorischer Aktivität. In humanen peripheren, polymorphnuklearen Leukozyten hemmt Acteosid die Bildung von Eicosatetraensäure-Derivaten (5-HET) und Leukotrien B₄, die beide an entzündlichen Prozessen maßgeblich beteiligt sind. Acteoside sowie die meisten Phenylethanoide besitzen aber auch interessante anti-oxidative Eigenschaften, da sie dosisabhängig die Fe²⁺/ADPinduzierte Lipidoxidation der Mitochondrial- und Liposomlipide aus der Rattenleber inhibieren können. Sie entwickeln dabei u.a. Radikalfängeraktivitäten auf Superoxidanion- und Hydroxylradikale. Für Acteoside konnte außerdem nachgewiesen werden, dass sie in der Lage sind, Chondroitinsulfat als wesentlichen Bestandteil der Knorpelmasse vor der Zersetzung durch Sauerstoffradikale zu schützen.

Die vorliegende Erfindung ist auf die eben genannten Pflanzenspezies keinesfalls beschränkt, sie bevorzugt diese aber auf Grund der einfachen Zugänglichkeit und Verarbeitbarkeit. Als Komponente II kommen aber auch entsprechend geeignete Zubereitungen von Pflanzen in Frage, wie sie bspw. in der Tabelle 1 dieser Beschreibung aufgelistet sind.

Die Breite der beanspruchten Zusammensetzung geht auch aus dem Gewichtsverhältnis hervor, zu dem die Komponenten I und II in der beanspruchten Zusammensetzung vorliegen können. Dieses ist nämlich keinesfalls auf ein bestimmtes Mischungsverhältnis beschränkt, sondern sie deckt vielmehr einen relativ breiten Bereich für die Komponenten ab, die insbesondere im Gewichtsverhältnis I : II wie 1 : 0,01 bis 0,5, bevorzugt 1 : 0,001 bis 1 und besonders bevorzugt 1 : 0,0001 bis 10 vorliegen sollten.

Wie der Wortlaut der Anspruchsfassung eindeutig belegt, ist die vorliegende Zusammensetzung nicht auf die beiden Komponenten I und II beschränkt, sondern sie muss diese beiden Komponenten "lediglich" enthalten. Aus diesem Grund sieht die vorliegende Erfindung auch eine Zusammensetzung vor, die neben diesen beiden Hauptkomponenten I und II noch weitere physiologisch aktive Komponenten, wie z.B. Glucosamin, Chondroitin, Hyaluronsäure, Methylsulfonylmethan und Kreatin oder deren geeignete Derivate enthalten.

Das Hauptauswahlkriterium für diese weiteren physiologisch aktiven Komponenten ist dabei deren positiver Einfluss auf in der Regel Entzündungserscheinungen, wie sie vor allem bei degenerativen Gelenkserkrankungen auftreten, bzw. deren Beitrag zu einer erfolgreichen Prävention, weshalb Komponenten im Vordergrund stehen, die ganz allgemein anti-oxidative und/oder anti-inflammatorische Wirkungsmechanismen unterstützen oder einen positiven Einfluss auf den Erhalt bzw. den Aufbau von Knorpelmasse oder die Knochenhaut ausüben.

Die vorliegende Erfindung sieht aber auch vor, dass die Zusammensetzung Formulierungshilfsmittel allgemeiner Art und/oder Stabilisierungsmittel, Füllstoffe, Aromastoffe, Farbstoffe und Süßungsmittel enthält, die der Konservierung der jeweils gewählten Darreichungsform dienen oder aber die Compliance erhöhen.

Im Vordergrund für die beanspruchte Zusammensetzung steht nicht deren Einsatz im traditionellen pharmazeutisch/medizinischen Bereich, sondern deren Verabreichung im Rahmen der Selbstindikation, wie sie die so genannten frei verkäuflichen OTC-Produkte darstellen. Aus diesem Grund sieht die vorliegende Erfindung bevorzugt vor, dass die beanspruchte Zusammensetzung in Form von Nahrungsergänzungsmitteln, funktionellen Lebensmitteln und kosmetischen Präparaten angeboten und appliziert wird, wobei insbesondere Tabletten, Kapseln, Dragees, Riegel, Granalien, Pulver, stabile Lösungen und Säfte besonders geeignete Darreichungsformen darstellen.

Wie bereits mehrfach erwähnt, soll die beanspruchte Zusammensetzung insbesondere degenerativen Gelenkserkrankungen förderlich sein. Die Erfindung beansprucht aus diesem Grund neben der eigentlichen Zusammensetzung auch deren Verwendung, wobei hierbei die Herstellung eines Mittels zur Vorbeugung und/oder Behandlung von entzündlichen und/oder degenerativen Erscheinungen, insbesondere mit chronischen Verlauf und besonders bevorzugt von Arthritis, Arthrosen und pathogener Angiogenese im Vordergrund stehen. Das so erhaltene Mittel eignet sich im Rahmen der vorliegenden Erfindung insbesondere für die Anwendung bei Berufs-, Freizeit- und Ausgleichssportlern, die einer verstärkten Gelenksbelastung, insbesondere im Kniebereich, ausgesetzt sind, aber auch bei älteren und rekonvaleszenten Personen und hier wiederum insbesondere bei Personen mit besonders beanspruchten und/oder beeinträchtigten Gelenksfunktionen.

Die beanspruchte Zusammensetzung und die damit hergestellten Mittel sind aufgrund ihrer kombinatorischen Wirkungsmöglichkeiten, die zum einen auf das Kollagen und zum anderen auf die anti-inflammatorischen/anti-oxidativen Eigenschaften der Komponente II zurückgehen, besonders für die Prävention von Gelenksüberbeanspruchungen und damit einhergehender Funktionseinschränkungen und Degenerationsprozessen geeignet, aber auch zu deren Behandlung.

In diesem Zusammenhang kann die tatsächliche Wirkung nicht darauf beschränkt gesehen werden, dass die Knorpelmasse bilanzmäßig zunimmt, sondern hauptsächlich darin, dass dem Abbau bzw. der Abnahme von Knorpelmasse vorgebeugt bzw. entgegengesteuert wird. Dabei kann es durchaus zur Förderung der Knorpelmassenbildung bzw. zu einer Neubildung der Knorpelmasse kommen, was aber nicht zwangsläufig mit einer insgesamt erhöhten Knorpelmasse einhergehen muss. Im Rahmen der vorliegenden Erfindung wird mit der beanspruchten Zusammensetzung somit vorrangig die Gesamt-Knorpelmasse bilanziert erhalten.

Die ebenfalls von der vorliegenden Erfindung beanspruchte spezielle Verwendung ist zusätzlich dadurch gekennzeichnet, dass das erhaltene Mittel in solchen Mengen verabreicht wird, die einer Tagesdosis an Komponente I von < 10 g, bevorzugt < 5 g entsprechen. Vorgesehen ist auch, dass das erhaltene Mittel kontinuierlich über einen Zeitraum von 2 Wochen bis 6 Monaten und bevorzugt von 3 Wochen bis 3 Monaten appliziert wird, was vorzugsweise oral erfolgen sollte.

Die mit der vorliegenden Erfindung erzielte Leistung kann somit auch darin gesehen werden, dass die Menge an zu verabreichendem Kollagen bzw. Gelatine nun von Mengen deutlich über 10 g/Tag auf signifikant kleinere Mengen als 10 g gesenkt werden kann, wobei zudem der bislang übliche Applikationszeitraum von > 8 Wochen ebenfalls deutlich verkürzt werden kann. Da trotz der deutlich gesenkten Tages-/Gesamtdosis bzgl. Komponente I deren Wirksamkeit in der erfindungsgemäßen Zusammensetzung keinesfalls verringert wurde, ist von einer deutlich synergistischen Wirksamkeitssteigerung auszugehen, die durch die erfindungsgemäße Kombination der Komponente I mit der Komponente II ausgeht, wobei der jeweilige genaue Wirkungsmechanismus allerdings noch nicht angegeben werden kann.

Die nachfolgende beispielhaft aufgeführte Studie belegt diesen positiven Effekt der beanspruchten Zusammensetzung.

### Beispiele

Im Rahmen einer doppelblind durchgeführten und Placebo-kontrollierten Studie wurde die Wirksamkeit eines als Nahrungsergänzungsmittel konzipierten Kombinationspräparats bei Patienten mit Kniegelenksarthrose überprüft. Die eingesetzte Zusammensetzung umfasste als alleinige Komponenten das Produkt "Arthred" (Kollagenhydrolysat von Degussa Food Ingredients GmbH) sowie den Spezialextrakt "Planox L" (von Anoxymer GmbH). "Arthred" besteht aus einem enzymatisch hydrolysierten Rinderkollagen und enthält ausschließlich kurzkettige Peptide mit einem Molekulargewicht von ca. 3 kDalton, wobei die Kettenlänge ca. 25 bis 30 Aminosäuren beträgt. "Planox L" wurde durch eine wässrig-alkoholische Extraktion aus dem getrockneten Kraut von Aloysia triphylla (L' Her.) O. Kuntze/Prit. (syn. Lippia citriodora H.B.K., Lippia triphylla (L.' Her.) O. Kuntze) gewonnen. Der Extrakt enthält Flavonoide und als Wirkstoff mindestens 10 Ges.-% des Phenylethanoids Acteosid.

Das Probandenklientel umfasste gehfähige, männliche und weibliche Patienten mit Kniegelenksarthrosen.

Ziel der Studie war die Optimierung des Allgemeinzustandes (Verbesserung der Symptomatik) leichter Kniegelenksarthrosen unter Beurteilung des Schmerzzustandes und des Funktionsstatus mittels. Lequesne-Index und WOMAC-Index (Western Ontario and McMaster Universities). Das Studiendesign war als doppelblinde, Placebo-kontrollierte und randomisierte Kurzzeitstudie bei Patienten mit Kniegelenksarthrosen ausgelegt und die Studie wurde als 4-armige Studie Placebo vs. Planox L vs. Arthred vs. Planox L + Arthred mit jeweils 25 Probanden und einer Gesamtstudiengröße von 100 Probanden bei jeweils oraler Applikation durchgeführt. Als Behandlungsdauer wurden 6 Monate pro Patient festgelegt.

Die Einschlusskriterien betrugen leichte Fälle von Kniegelenksarthrosen; Zustand nach Arthroskopie ohne tiefreichende Knorpeldefekte; uneingeschränkte Altersgruppen; männlich und weibliches Geschlecht.

Als Ausschlusskriterien wurden Erkrankungen des rheumatischen Formenkreises; Infektionen (insbesondere Gelenksinfektionen); schwere Fälle von Kniegelenksarthrosen; Band-, Knorpelersatz- und Gelenk-OP's (außer Arthroskopie); NSAR/Kortikoid/Antibiotika-Medikationen (außer Lipidsenker, Antihypertonika, Antiarrhythmika); Verabreichung von Vitaminen, Mineralstoffen und Phytotherapeutika, insbesondere Pflanzenextrakten aus der Teufelskralle und Artischocke sowie Wobenzym-Hyaluronsäure-Injektionen definiert.

Die Studienmedikation verlief wie folgt:

| | |
|---|---|
| Planox L: | 1 x täglich 1 g Planox L (10 % Acteosid |
| | (=100 mg Acteosid täglich) + 5 g Cellulose) |
| Arthred: | 1 x täglich 5 g Kollagenhydrolysat |
| Planox L + Arthred: | 1 x täglich 1 g Planox L + 5 g Kollagenhydrolysat |
| Placebo: | 1 x täglich 5 g mikrokristalline Cellulose |

Bei Studienbeginn wurden von jedem Patienten der BSG-Wert und die radiologischen Daten (Röntgenbilder) erfasst. Ggf. wurde durch Punktion Synovial-Flüssigkeit aus dem Kniegelenk gewonnen. Ebenso wurde der Schmerzzustand und der Funktionsstatus des Kniegelenks mit Hilfe der entsprechenden Indizes bestimmt. Darüber hinaus erfolgte eine 6-Wochen-Kontrolle des Schmerzzustandes und des Funktionsstatus des Kniegelenks, d.h. in Woche 6, 12, 18 und 24 nach Studienbeginn.

### Ergebnisse:

Arthred und Planox L zeigten bei alleiniger Gabe (Vergleichsversuche) keine bzw. eine nicht-signifikante Verbesserung. Mit der erfindungsgemäßen Zusammensetzung, bestehend aus einer Kombination von Arthred + Planox L, trat überraschenderweise eine synergistische Wirkung auf, die über die Wirkung der Einzelsubstanzen deutlich hinausging. Bei Gabe der kombinierten Zusammensetzung verminderte sich der Schmerzzustand signifikant und der Funktionsstatus des Kniegelenks wurde deutlich erhöht.

## Patentansprüche

1. Physiologisch aktive Zusammensetzung auf Kollagenbasis, enthaltend als aktive Komponente I enzymatisch hydrolysiertes Kollagen sowie eine aktive Komponente II vom nicht-Vitamin-Typ mit anti-oxidativen und/oder antiinflammatorischen Eigenschaften, **dadurch gekennzeichnet, dass** die Komponente II mindestens ein Phenylethanoid enthält, wobei das Phenylethanoid mindestens ein Acteosid und/oder Isoacteosid enthält.

2. Zusammensetzung nach Anspruch 1, **dadurch gekennzeichnet, dass** die Komponente I ein mittleres Molekulargewicht von 1 bis 10, besonders bevorzugt von 2 bis 6 und ganz besonders bevorzugt von 2 bis 4 kDalton aufweist.

3. Zusammensetzung nach einem der Ansprüche 1 oder 2, **dadurch gekennzeichnet, dass** die Komponente I tierischen Ursprungs ist.

4. Zusammensetzung nach einem der Ansprüche 1 bis 3, **dadurch gekennzeichnet, dass** sie als Komponente I ein kaltwasserlösliches Kollagen enthält.

5. Zusammensetzung nach einem der Ansprüche 1 bis 4, **dadurch gekennzeichnet, dass** die Komponente II eine Komponente biologischen Ursprungs ist, bevorzugt auf Basis einer Fermentationsbrühe und/oder eines Extrakts, der besonders bevorzugt pflanzlichen Ursprungs ist.

6. Zusammensetzung nach Anspruch 5, **dadurch gekennzeichnet, dass** es sich bei der Komponente II um einen Extrakt, ein Lyophilisat und/oder eine Fraktion handelt, insbesondere von einem pflanzlichen Vertreter von Plantago spec., wie z.B. P. mayor, P. media, P. lanceolata, Aloysia triphylla, Lippia triphylla und/oder Lippia citriodora.

7. Zusammensetzung nach Anspruch 6, **dadurch gekennzeichnet, dass** die Komponente II zusätzlich einen COX-2 Inhibitor, besonders bevorzugt Alpha-Säuren und Beta-Säuren, Apigenin, Baicalein, Berberin, Cinnamaldehyd, Cirsilineol, Curcumin, Eugenol, Kaempferol, Oleandsäure, Quercetin, Resveratrol, Salicylsäure, Ursolinsäure, enthält.

8. Zusammensetzung nach einem der Ansprüche 1 bis 7, **dadurch gekennzeichnet, dass** sie die Komponenten I und II im Gewichtsverhältnis I : II wie 1 : 0,0001 bis 10 enthält.

9. Zusammensetzung nach einem der Ansprüche 1 bis 8, **dadurch gekennzeichnet, dass** sie neben den Hauptkomponenten I und II weitere physiologisch aktive Komponenten, wie z.B. Glucosamin, Chondroitin, Hyaluronsäure, Methylsulfonylmethan und Kreatin oder deren geeignete Derivate, und/oder Formulierungshilfsmittel, Stabilisierungsmittel, Füllstoffe, Aromastoffe, Farbstoffe und Süßungsmittel enthält.

10. Zusammensetzung nach einem der Ansprüche 1 bis 9 in Form von Nahrungsergänzungsmitteln, funktionellen Lebensmitteln und kosmetischen Präparaten, insbesondere in Form von Tabletten, Kapseln, Dragees, Riegeln, Granalien, Pulvern, stabilen Lösungen und Säften.

11. Verwendung der Zusammensetzung nach einem der Ansprüche 1 bis 10 zur Herstellung eines Mittels zur Vorbeugung und/oder Behandlung von entzündlichen und/oder degenerativen Erscheinungen, insbesondere mit chronischem Verlauf und besonders bevorzugt von Arthritis, Arthrosen und pathogener Angiogenese.

12. Verwendung nach Anspruch 11 bei Berufs-, Freizeit- und Ausgleichssportlern, älteren und rekonvaleszenten Personen, insbesondere bei Personen mit besonders beanspruchten und/oder beeinträchtigten Gelenksfunktionen.

13. Verwendung nach Anspruch 12, **dadurch gekennzeichnet, dass** das erhaltene Mittel in Mengen verabreicht wird, welche einer Tagesdosis an Komponente I von < 10 g entsprechen.

14. Verwendungen nach einem der Ansprüche 11 bis 13, **dadurch gekennzeichnet, dass** das erhaltene Mittel kontinuierlich über einen Zeitraum von 2 Wochen bis 6 Monaten und bevorzugt von 3 Wochen bis 3 Monaten, vorzugsweise oral, appliziert wird.

## Claims

1. A physiologically active composition based on collagen, containing enzymatically hydrolyzed collagen as active component I and an active component II of the non-vitamin type with anti-oxidative and/or anti-inflammatory properties, **characterized in that** said component II contains at least one phenylethanoid, wherein said phenylethanoid contains at least one acteoside and/or isoacteoside.

2. A composition according to claim 1, **characterized in that** said component I has a mean molecular weight of 1 to 10, particularly preferred of 2 to 6 and especially preferred of 2 to 4 kDaltons.

3. A composition according to any one of claims 1 or 2, **characterized in that** said component I is of animal origin.

4. A composition according to any one of claims 1 to 3, **characterized in that** it contains a collagen that is soluble in cold water as component I.

5. A composition according to any one of claims 1 to 4, **characterized in that** said component II is a component of biological origin, preferably based on a fermentation broth and/or an extract which, particularly preferred, is of plant origin.

6. A composition according to claim 5, **characterized in that** said component II is an extract, a lyophilized culture and/or a fraction, in particular from a plant species of Plantago spec., such as e.g. P. mayor, P. media, P. lanceolata, Aloysia triphylla, Lippia triphylla and/or Lippia citriodora.

7. A composition according to claim 6, **characterized in that** said component II additionally contains a COX-2 inhibitor, particularly preferred alpha acids and beta acids, apigenin, baicalein, berberine, cinnamaldehyde, cirsilineol, curcumin, eugenol, kaempferol, oleanolic acid, quercetin, resveratrol, salicylic acid, ursolic acid.

8. A composition according to any one of claims 1 to 7, **characterized in that** it contains said components I and II in a weight ratio of I : II being 1 : 0.0001 to 10.

9. A composition according to any one of claims 1 to 8, **characterized in that**, in addition to the main components I and II, it contains further physiologically active components, such as e.g. glucosamine, chondroitin, hyaluronic acid, methylsulphonylmethane and creatine or suitable derivates thereof, and/or formulation auxiliaries, stabilizers, fillers, flavourings, colourants and sweeteners.

10. A composition according to any one of claims 1 to 9 in the form of dietary supplements or functional food and cosmetic preparations, in particular in the form of tablets, capsules, dragées, bars, granules, powders, stable solutions and juices.

11. The use of the composition according to any one of claims 1 to 10 for manufacturing a product for the prevention or treatment of inflammatory and/or degenerative diseases, in particular of a chronic nature and particularly preferred of arthritis, osteoarthritis and pathological angiogenesis.

12. The use according to claim 11 for professional and amateur sportspeople as well as people doing recreational sports, elderly and convalescent people, in particular people whose joint functions are subjected to high stress and/or are limited.

13. The use according to claim 12, **characterized in that** the product obtained is administered in amounts corresponding to a daily dose of said component I of < 10 g.

14. Uses according to any one of claims 11 to 13, **characterized in that** the product obtained is applied, preferably orally, during a continuous period of 2 weeks to 6 months and preferably of 3 weeks to 3 months.

## Revendications

1. Composition physiologiquement active à base de collagène, contenant un collagène hydrolysé de manière enzymatique en tant que composant actif I ainsi qu'un composant actif II de type non vitamine avec des propriétés anti-oxydantes et/ou anti-inflammatoires, **caractérisée en ce que** le composant II contient au moins un phényléthanoïde, le phényléthanoïde contenant au moins un actéoside et/ ou un isoactéoside.

2. Composition selon la revendication 1, **caractérisée en ce que** le composant I présente un poids moléculaire moyen de 1 à 10, de manière particulièrement préférée de 2 à 6 et de manière plus particulièrement préférée de 2 à 4 kDalton.

3. Composition selon l'une quelconque des revendications 1 ou 2, **caractérisée en ce que** le composant I est d'origine animale.

4. Composition selon l'une quelconque des revendications 1 à 3, **caractérisée en ce qu'**elle contient un collagène soluble dans l'eau froide en tant que composant I.

5. Composition selon l'une quelconque des revendications 1 à 4, **caractérisée en ce que** le composant II est un composant d'origine biologique, de manière préférée à base d'une boue de fermentation et/ou d'un extrait qui est de manière particulièrement préférée d'origine végétale.

6. Composition selon le revendication 5, **caractérisée en ce que** le composant II est un extrait, un lyophilisat et/ou une fraction, en particulier d'un représentant végétal de l'espèce Plantago, comme par exemple P. mayor, P. media, P. lanceolata, Aloysia triphylla, Lippia triphylla et/ou Lippia citriodora.

7. Composition selon la revendication 6, **caractérisée en ce que** le composant II contient de manière supplémentaire un inhibiteur de COX-2, de manière particulièrement préférée des alpha-acides et des béta-acides, apigénine, baicaline, berbérine, cinnamaldéhyde, cirsilinéol, curcumine, acide eugénique, huile de camphre, acide oléanolique, quercétine, resvératrol, acide salicylique, acide ursolinique.

8. Composition selon l'une quelconque des revendications 1 à 7, **caractérisée en ce qu'**elle contient les composants I et II en un rapport en poids I:II de 1:0,0001 à 10.

9. Composition selon l'une quelconque des revendications 1 à 8, **caractérisée en ce qu'**elle contient d'autres composants physiologiquement actifs en plus des composants principaux I et II, comme par exemple glucosamine, chondroïtine, acide hyaluronique, méthylsulfonylméthane et créatine ou leurs dérivés appropriés, et/ou des auxiliaires de formulation, des agents de stabilisation, des matières de charge, des substances aromatiques, des colorants et des édulcorants.

10. Composition selon l'une quelconque des revendications 1 à 9 sous forme de compléments alimentaires, d'aliments fonctionnels et de préparations cosmétiques, en particulier sous la forme de tablettes, de capsules, de dragées, de barres, de granules, de poudres, de solutions stables et de sirops.

11. Utilisation de la composition selon l'une quelconque des revendications 1 à 10 pour la préparation d'un produit destiné à éviter et/ou traiter des symptômes inflammatoires et/ou dégénératifs, en particulier présentant une évolution chronique et de manière particulièrement préférée associés à l'arthrite, l'arthrose et l'angiogenèse pathogène.

12. Utilisation selon la revendication 11 auprès de sportifs professionnels, amateurs et occasionnels, de personnes âgées et convalescentes, en particulier de personnes présentant des fonctions articulaires particulièrement sollicitées et/ou endommagées.

13. Utilisation selon la revendication 12, **caractérisée en ce que** le produit reçu est administré en des quantités qui correspondent à une posologie journalière de composant I < 10 g.

14. Utilisations selon l'une quelconque des revendications 11 à 13, **caractérisée en ce que** le produit reçu est appliqué en continu sur une durée de 2 semaines à 6 mois et de manière préférée de 3 semaines à 3 mois, de préférence de manière orale.
